(19) Europäisches Patentamt
European Patent Office
Office européen des brevets

(11) **EP 4 722 225 A1**

(12) **EUROPEAN PATENT APPLICATION**

(43) Date of publication:
**08.04.2026 Bulletin 2026/15**

(21) Application number: **24306608.1**

(22) Date of filing: **01.10.2024**

(51) International Patent Classification (IPC):
***C07F 17/00*** *(2006.01)* ***C07C 1/32*** *(2006.01)*
***C07F 7/08*** *(2006.01)* ***C07F 5/02*** *(2006.01)*

(52) Cooperative Patent Classification (CPC):
(C-Sets available)
**C07F 17/00; C07C 1/321; C07F 7/0807;**
C07C 2602/08; C07F 5/025; C07F 7/0832 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **TotalEnergies OneTech**
**92400 Courbevoie (FR)**

(72) Inventors:
- **CASARRUBIOS, Lorentz**
  **7181 Seneffe (BE)**
- **COUSTHAM, Thomas**
  **7670 Rogerville (FR)**
- **WELLE, Alexandre**
  **7181 Seneffe (BE)**

(74) Representative: **De Clercq & Partners**
**Edgard Gevaertdreef 10a**
**9830 Sint-Martens-Latem (BE)**

# (54) SYNTHESIS OF RAC-DIMETHYLSILYLENEBIS(2-METHYL-4-[3',5'-DI-T-BUTYLPHENYL] INDENYL) ZIRCONIUM DICHLORIDE, INTERMEDIATES THEREOF, AND USES THEREOF

(57) The present invention relates to a process for the preparation of 4-(3',5'-di-t-butylphenyl)-2-methyl-1H-indene (Cpd-3),

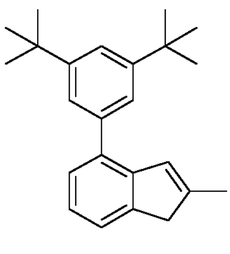

Cpd-3

the process comprising the steps as defined in the claims.

The present invention also relates to a process for the preparation of rac-dimethylsilylenebis(2-methyl-4-[3',5'-di-t-butylphenyl]indenyl) zirconium dichloride (rMet1),

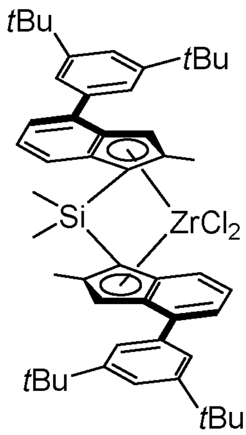

rMet1

the process comprising the steps as defined in the claims.

The present invention also relates to a catalyst composition comprising: rac-dimethylsilylenebis(2-methyl-4-[3',5'-di-t-butylphenyl]indenyl) zirconium dichloride (rMet1); a titanated solid oxide support; at least one optional activator; and at least one optional cocatalyst. The present invention also relates to an olefin polymerization process, the process comprising: contacting at least one catalyst composition as defined in the claims, with an olefin monomer, optionally hydrogen, and optionally one or more olefin comonomers; and polymerizing the monomer, and the optionally one or more olefin comonomers, in the presence of the at least one catalyst composition, and optional hydrogen, thereby obtaining an olefin polymer.

(52) Cooperative Patent Classification (CPC): (Cont.)

C-Sets
**C07C 1/321, C07C 13/465**

## Description

### Field of invention

The invention relates to the synthesis of metallocene compound rac-dimethylsilylenebis(2-methyl-4-[3',5'-di-t-butylphenyl]indenyl) zirconium dichloride and intermediates thereof. The invention further relates to catalyst compositions comprising rac-dimethylsilylenebis(2-methyl-4-[3',5'-di-t-butylphenyl]indenyl) zirconium dichloride and uses thereof in polymerization reactions. This invention also relates to new propylene polymers obtained from said polymerization reactions.

### Background of the invention

In the frame of commodity polymers made out with metallocene, there is a need to have resins with enhanced properties. While polypropylene resins made with Ziegler-Natta catalysts have good activity, metallocene catalyst affords products having narrow molecular weight distribution, with very high fluidity index without any additives. Nevertheless, the lower activity and lower melting temperature of polypropylene resin made with metallocene catalysts are the major drawbacks.

Currently, polypropylene resins made with Ziegler-Natta catalyst have a good activity and high melting temperature resins but need addition of peroxide to have high fluidity index. Addition of peroxide in fortunately lead to volatile organic compounds (VOC). Finding a balance between said physical and mechanical properties presents a significant challenge within the art.

There is thus still a need for metallocene catalysts and compositions thereof that have a stable catalyst activity and can be used to produce polypropylene with desirable physical and mechanical properties.

### Summary of the invention

In a first aspect, the present invention provides a process for the preparation of 4-(3',5'-di-*t*-butylphenyl)-2-methyl-1H-indene (Cpd-3),

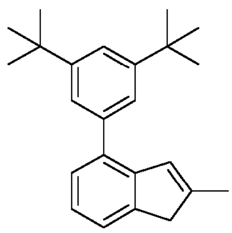

Cpd-3

the process comprising the step of:

coupling a compound of Formula (I) with a compound of Formula (II) by means of a Suzuki coupling reaction (preferably via Suzuki-Miyaura coupling reaction) thereby generating 4-(3',5'-di-*t*-butylphenyl)-2-methyl-1H-indene (Cpd-3),

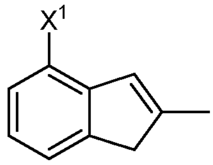

I,

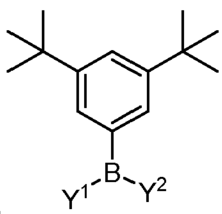

II,

wherein $X^1$ is selected from the group comprising halogen, tosylate, mesylate, triflate, nonaflate, and pentafluorophenol; and

$Y^1$, $Y^2$ are each independently selected from the group comprising hydroxyl, alkyl, alkenyl, alkynyl, and alkoxy, or $Y^1$ and $Y^2$ form together with the boron atom they are bound to a mono-, bi- or polycyclic ring.

In a second aspect, the present invention provides a process for the preparation of bis(4-(3,5-di-tert-butylphenyl)-2-methyl-1H-inden-1-yl)dimethylsilane (Cpd-4),

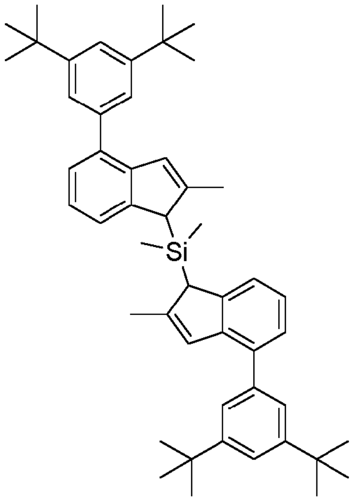

Cpd-4

the process comprising the steps of:

a) preparing 4-(3',5'-di-*t*-butylphenyl)-2-methyl-1H-indene (Cpd-3) by means of the process according to the first aspect of the invention; and

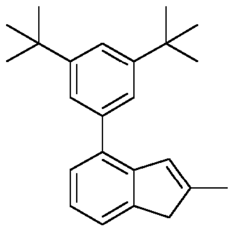

Cpd-3

b) deprotonating Cpd-3 using at least one base to form an indenide anion, followed by c) reaction with $Me_2SiX^2{}_2$, optionally in the presence of a catalyst, thereby forming bis(4-(3,5-di-tert-butylphenyl)-2-methyl-1H-inden-1-yl) dimethylsilane (Cpd-4), wherein each $X^2$ is independently selected from the group consisting of halogen, tosylate, mesylate, triflate, nonaflate, and pentafluorophenol.

In a third aspect, the present invention provides a process for the preparation of rac-dimethylsilylenebis(2-methyl-4-[3',5'-di-t-butylphenyl]indenyl) zirconium dichloride (*r*Met1),

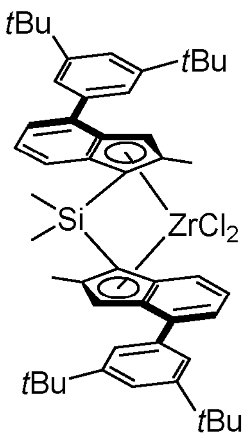

*r*Met1

the process comprising the steps of:

(a) preparing bis(4-(3,5-di-tert-butylphenyl)-2-methyl-1H-inden-1-yl)dimethylsilane (Cpd-4) by means of the process according to the second aspect;

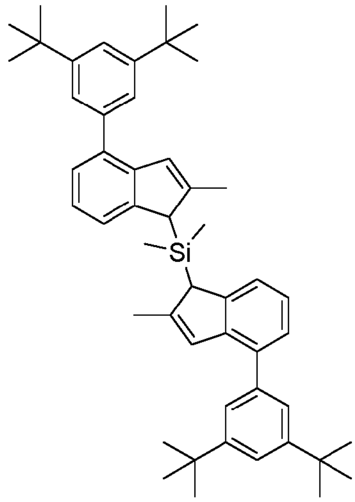
Cpd-4

(b) deprotonating Cpd-4 using at least one base to form indenide dianion, followed by (c) reaction with $ZrCl_4$, thereby forming dimethylsilylenebis(2-methyl-4-[3',5'-di-t-butylphenyl]indenyl) zirconium dichloride; and

(d) crystallizing dimethylsilylenebis(2-methyl-4-[3',5'-di-t-butylphenyl]indenyl) zirconium dichloride in toluene and isolating rac-dimethylsilylenebis(2-methyl-4-[3',5'-di-t-butylphenyl]indenyl) zirconium dichloride (rMet1).

The present synthesis provides a new alternative way of preparing rac-dimethylsilylenebis(2-methyl-4-[3',5'-di-t-butylphenyl]indenyl) zirconium dichloride. The synthesis is amenable to large scale production of said catalyst.

In a fourth aspect, the present invention also provides a catalyst composition comprising:

- rac-dimethylsilylenebis(2-methyl-4-[3',5'-di-*t*-butylphenyl]indenyl) zirconium dichloride, for example as obtained according to the process according to the third aspect of the invention;
- a titanated solid oxide support, preferably a titanated silica support;
- at least one optional activator; and at least one optional co-catalyst.

In a sixth aspect, the present invention also provides a process for preparing a catalyst composition according to the fourth aspect of the invention, the process comprising the steps of:

- providing rac-dimethylsilylenebis(2-methyl-4-[3',5'-di-*t*-butylphenyl]indenyl) zirconium dichloride, preferably as prepared using the process according to third aspect of the invention;
- contacting the rac-dimethylsilylenebis(2-methyl-4-[3',5'-di-*t*-butylphenyl]indenyl) zirconium dichloride with a titanated solid oxide support, thereby obtaining the catalyst composition.

The present invention also provides an olefin polymerization process, the process comprising: contacting at least one catalyst composition according to the fourth aspect of the invention, or obtained or obtainable by carrying out the process according to the sixth aspect of the invention, with an olefin monomer, optionally hydrogen, and optionally one or more olefin comonomers; and polymerizing the monomer, and the optionally one or more olefin comonomers, in the presence of the at least one catalyst composition, and optional hydrogen, thereby obtaining an olefin polymer.

The present metallocene compositions also provides polypropylene resins with unique molecular architectures, high melting temperature, and with melt indexes which can be tailored according to the application sought.

The independent and dependent claims set out particular and preferred features of the invention. Features from the dependent claims may be combined with features of the independent or other dependent claims as appropriate.

The present invention will now be further described. In the following passages, different aspects of the invention are defined in more detail. Each aspect so defined may be combined with any other aspect or aspects unless clearly

indicated to the contrary. In particular, any feature or statement indicated as being preferred or advantageous may be combined with any other features or statements indicated as being preferred or advantageous.

## Brief description of the figures

Figure 1 represents a graph plotting the melt flow index $MI_{105}$ as a function of melt flow index $MI_2$ measured for polypropylene resins.

Figure 2 represents a graph plotting the $^{13}C\{^1H\}$ NMR spectrum of a metallocene catalyzed propylene-ethylene copolymer containing about 5 % by weight of ethylene.

Figure 3 represents a graph plotting the $^1H$ NMR spectrum of bis(4-(3,5-di-tert-butylphenyl)-2-methyl-1H-inden-1-yl) dimethylsilane (Cpd-4).

Figure 4 represents a graph plotting the $^1H$ NMR spectrum of rac-dimethylsilylenebis(2-methyl-4-[3',5'-di-*t*-butylphenyl]indenyl) zirconium dichloride (rMet1).

Figures 5, 6, 7, and 8 represent graphs plotting the molecular weight distribution (weight fraction (area normalized) as a function of logarithm of molecular weight) (GPC traces) of polypropylene homopolymers A, B, C, and D, respectively.

Figures 9, 10, and 11 represent graphs plotting the molecular weight distribution (weight fraction (area normalized) as a function of logarithm of molecular weight) of polypropylene copolymers E, F, and G, respectively.

## Detailed description of the invention

Before the present synthesis, catalysts, compositions, compounds, polymers, processes, articles, and uses encompassed by the invention are described, it is to be understood that this invention is not limited to particular synthesis, catalysts, compositions, compounds, polymers, processes, articles, and uses described, as such catalysts, compositions, compounds, polymers, processes, articles, and uses may, of course, vary. It is also to be understood that the terminology used herein is not intended to be limiting, since the scope of the present invention will be limited only by the appended claims.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. When describing the compounds, processes, articles, and uses of the invention, the terms used are to be construed in accordance with the following definitions, unless the context dictates otherwise.

The discussion of the background to the invention herein is included to explain the context of the invention. This is not to be taken as an admission that any of the material referred to was published, known, or part of the common general knowledge in any country as of the priority date of any of the claims. Throughout this disclosure, various publications, patents, and published patent specifications are referenced by an identifying citation. All documents cited in the present specification are hereby incorporated by reference in their entirety. In particular, the teachings or sections of such documents herein specifically referred to are incorporated by reference.

As used herein, the singular forms "a", "an", and "the" include both singular and plural referents unless the context clearly dictates otherwise. By way of example, "a polymer" means one polymer or more than one polymer.

The terms "comprising", "comprises" and "comprised of" as used herein are synonymous with "including", "includes" or "containing", "contains", and are inclusive or open-ended and do not exclude additional, non-recited members, elements, or method steps. The terms also encompass "consisting of" and "consisting essentially of", which enjoy well-established meanings in patent terminology.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

As used herein, the term "and/or" when used in a list of two or more items, means that any one of the listed items can be employed by itself or any combination of two or more of the listed items can be employed. For example, if a list is described as comprising group A, B, and/or C, the list can comprise A alone, B alone, C alone, A and B in combination, A and C in combination, B and C in combination, or A, B, and C in combination.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" or "in a particular embodiment" in various places throughout this specification are not necessarily all referring to the same embodiment, but may. Furthermore, the particular features, structures or characteristics may be combined in any suitable manner, as would

be apparent to a person skilled in the art from this disclosure, in one or more embodiments. Furthermore, while certain embodiments described herein include some but not other features included in other embodiments, combinations of features of different embodiments are meant to be within the scope of the invention, and form different embodiments, as would be understood by those in the art. For example, in the following claims and statements, any of the embodiments can be used in any combination.

The recitation of numerical ranges by endpoints includes all integer numbers and, where appropriate, fractions subsumed within that range (e.g., 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g., from 1.0 to 5.0 includes both 1.0 and 5.0). Any numerical range recited herein is intended to include all sub-ranges subsumed therein. This applies to numerical ranges irrespective of whether they are introduced by the expression "from... to..." or the expression "between... and..." or another expression.

The terms "wt.%," "vol%", or "mol%" refers to a weight percentage of a component, a volume percentage of a component, or molar percentage of a component, respectively, based on the total weight, the total volume of material, or total moles, which includes the component. Whenever the term "substituted" is used herein, it is meant to indicate that one or more hydrogen atoms on the atom indicated in the expression using "substituted" is replaced with a selection from the indicated group, provided that the indicated atom's normal valence is not exceeded, and that the substitution results in a chemically stable compound, i.e., a compound that is sufficiently robust to survive isolation from a reaction mixture.

The term "halo" or "halogen" as a group or part of a group is generic for fluoro, chloro, bromo, iodo.

The term "alkyl" as a group or part of a group, refers to a hydrocarbyl group of formula $C_nH_{2n+1}$ wherein n is a number greater than or equal to 1. Alkyl groups may be linear or branched and may be substituted as indicated herein. Generally, alkyl groups of this invention comprise from 1 to 20 carbon atoms, preferably from 1 to 10 carbon atoms, preferably from 1 to 6 carbon atoms, more preferably from 1 to 4 carbon atoms. When a subscript is used herein following a carbon atom, the subscript refers to the number of carbon atoms that the named group may contain. For example, the term "$C_{1-20}$alkyl", as a group or part of a group, refers to a hydrocarbyl group of formula $-C_nH_{2n+1}$ wherein n is a number ranging from 1 to 20. Thus, for example, "$C_{1-8}$alkyl" includes all linear or branched alkyl groups having from 1 to 8 carbon atoms, and thus includes methyl, ethyl, n-propyl, i-propyl, butyl and its isomers (e.g., n-butyl, i-butyl and t-butyl); pentyl and its isomers, hexyl and its isomers, etc. A "substituted alkyl" refers to an alkyl group substituted with one or more substituent(s) (for example 1 to 3 substituent(s), for example 1, 2, or 3 substituent(s)) at any available point of attachment.

When the suffix "ene" is used in conjunction with an alkyl group, i.e., "alkylene", this is intended to mean the alkyl group as defined herein having two single bonds as points of attachment to other groups. As used herein, the term "alkylene" also referred as "alkanediyl", by itself or as part of another substituent, refers to alkyl groups that are divalent, i.e., with two single bonds for attachment to two other groups. Alkylene groups may be linear or branched and may be substituted as indicated herein. Non-limiting examples of alkylene groups include methylene ($-CH_2-$), ethylene ($-CH_2-CH_2-$), methylmethylene ($-CH(CH_3)-$), 1-methyl-ethylene ($-CH(CH_3)-CH_2-$), n-propylene ($-CH_2-CH_2-CH_2-$), 2-methylpropylene ($-CH_2-CH(CH_3)-CH_2-$), 3-methylpropylene ($-CH_2-CH_2-CH(CH_3)-$), n-butylene ($-CH_2-CH_2-CH_2-CH_2-$), 2-methylbutylene ($-CH_2-CH(CH_3)-CH_2-CH_2-$), 4-methylbutylene ($-CH_2-CH_2-CH_2-CH(CH_3)-$), pentylene and its chain isomers, hexylene and its chain isomers.

The term "alkenyl" as a group or part of a group, refers to an unsaturated hydrocarbyl group, which may be linear, or branched, comprising one or more carbon-carbon double bonds.

Generally, alkenyl groups of this invention comprise from 3 to 20 carbon atoms, preferably from 3 to 10 carbon atoms, preferably from 3 to 8 carbon atoms. When a subscript is used herein following a carbon atom, the subscript refers to the number of carbon atoms that the named group may contain. Examples of $C_{3-20}$alkenyl groups are ethenyl, 2-propenyl, 2-butenyl, 3-butenyl, 2-pentenyl and its isomers, 2-hexenyl and its isomers, 2,4-pentadienyl, and the like. The term "alkynyl" as a group or part of a group, refers to a branched or straight chain hydrocarbon comprising at least one site (usually 1 to 3, preferably 1) of unsaturation, namely a $sp^1$ carbon-$sp^1$ carbon triple bond. In particular embodiments, the term alkynyl refers to $C_{2-12}$ alkynyl ($C_{2-12}$ hydrocarbons), preferably to $C_{2-9}$ alkynyl ($C_{2-9}$ hydrocarbons) yet more preferably to $C_{2-6}$ alkynyl ($C_{2-6}$ hydrocarbons) as further defined herein above with at least one site (usually 1 to 3, preferably 1) of unsaturation, namely at least one $sp^1$ carbon-$sp^1$ carbon triple bond. Examples of alkynyl include but are not limited to: ethynyl ($-C\equiv CH$), 3-ethyl-cyclohept-1-ynylene, and 1-propynyl (propargyl, $-CH_2C\equiv CH$).

The term "alkoxy" or "alkyloxy", as a group or part of a group, refers to a group having the formula $-OR^b$ wherein $R^b$ is alkyl as defined herein above. Non-limiting examples of suitable alkoxy include methoxy, ethoxy, propoxy, isopropoxy, butoxy, isobutoxy, sec-butoxy, tert-butoxy, pentyloxy and hexyloxy.

The term "hydroxyl" or "hydroxy" as used herein refers to the group -OH.

Preferred statements (features) and embodiments of the compositions, processes, polymers, articles, and uses of this invention are set herein below. Each statement and embodiment of the invention so defined may be combined with any other statement and/or embodiment, unless clearly indicated to the contrary. In particular, any feature indicated as being preferred or advantageous may be combined with any other features or statements indicated as being preferred or advantageous. Hereto, the present invention is in particular captured by any one or any combination of one or more of the

below numbered statements and embodiments, with any other aspect and/or embodiment.

1. A process for the preparation of 4-(3',5'-di-*t*-butylphenyl)-2-methyl-1H-indene (Cpd-3),

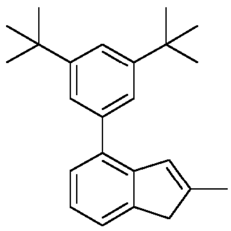

Cpd-3

the process comprising the step of:

coupling a compound of Formula (I) with a compound of Formula (II) by means of a Suzuki coupling reaction (preferably via Suzuki-Miyaura coupling reaction) thereby generating 4-(3',5'-di-*t*-butylphenyl)-2-methyl-1H-indene (Cpd-3),

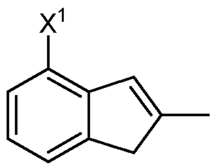

I,

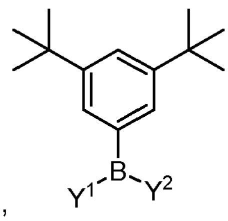

II,

wherein $X^1$ is selected from the group comprising halogen, tosylate, mesylate, triflate, nonaflate, and penta-fluorophenol; and

$Y^1$, $Y^2$ are each independently selected from the group comprising hydroxyl, alkyl, alkenyl, alkynyl, and alkoxy, or $Y^1$ and $Y^2$ form together with the boron atom they are bound to a mono-, bi- or polycyclic ring.

2. The process according to statement 1, wherein said Suzuki coupling is performed in the presence of at least one metal catalyst, wherein the metal catalyst comprises palladium, nickel, or copper, or a combination thereof, preferably said metal catalyst is at least one palladium catalyst.

3. The process according to any one of statements 1-2, wherein said coupling is performed in the presence of at least one palladium catalyst, wherein the catalyst is a palladium compound with at least one organophosphine ligand, or the catalyst is a palladium precatalyst complex.

4. The process according to any one of statements 1-3, wherein said coupling is performed in the presence of at least one palladium compound with an organophosphine ligand.

5. The process according to any one of statements 1-4, wherein said coupling is performed in the presence of at least one palladium compound with at least one organophosphine ligand, wherein the palladium compound is selected from the group comprising $Pd(OAc)_2$, $Pd(PPh_3)_4$, $Pd(TFA)_2$, $Pd(MeCN)_2Cl_2$, $Pd_2(dba)_3$, $PdBr_2$, and a combination of any two or more thereof, preferably $Pd(OAc)_2$.

6. The process according to any one of statements 1-5, wherein said coupling is performed in the presence of at least one palladium compound with at least one organophosphine ligand, for example at least one organophosphine selected from the group consisting of SPhos, $PPh_3$, XPhos, APhos, CPhos, RuPhos, cataCXium, DavePhos, JohnPhos, MePhos, XantPhos, $tBuPPh_2$ and combinations thereof, preferably SPhos, XPhos, APhos, CPhos, RuPhos, $PPh_3$, more preferably SPhos, XPhos, $PPh_3$, most preferably SPhos.

7. The process according to any one of statements 1-6, wherein said coupling is performed in the presence of at least one palladium catalyst, wherein the palladium catalyst is at least one palladium precatalyst complex, wherein the palladium precatalyst complex is selected from the group comprising SPhos Pd G2, XPhos Pd G2, P(Cy3)Pd G3,

APhos Pd G3, cataC-Pd G2 and cataC-Pd G3.

8. The process according to any one of statements 1-7, wherein said coupling is performed in the presence of at least one palladium compound and at least one organophosphine ligand, wherein the ligand:palladium compound molar ratio is from 1:1 to 3:1, preferably 2:1 to 1:1.

9. The process according to any one of statements 1-8, wherein said coupling is performed in the presence of at least one base, preferably selected from the group comprising $K_3PO_4$, LiOH, CsF, KF, NaF, LiF, AgF, $MgF_2$, NaOtBu, KOtBu, NaOH, KOH, MeONa, MeOK, $Cs_2CO_3$, $K_2CO_3$, $KHCO_3$, $Na_2CO_3$ and NaHCOs; preferably the base is $K_3PO_4$.

10. The process according to any one of statements 1-9, wherein said coupling is performed in at least one solvent, preferably in at least one organic solvent, for example in a mixture of water and at least one organic solvent.

11. The process according to any one of statements 1-10, wherein said coupling is performed in at least one solvent, preferably in at least one organic solvent, for example in a mixture of water and at least one organic solvent, preferably wherein the at least one organic solvent is selected from the group comprising THF, MeTHF, dioxane, toluene, benzene, DMF, DMSO, DMAc, IPA, MeOH and EtOH; preferably the solvent is THF, preferably the coupling is performed in a mixture of water and organic solvent, wherein the ratio of water to the organic solvent is 1/1 to 1/100; particularly the ratio is 1/1 to 1/40, preferably 1/1 to 1/10, preferably 1:1 to 1/5.

12. The process according to any one of statements 1-11, wherein said reaction is quenched with at least one acid solution; preferably wherein said acid solution is selected from the group comprising HCl, HBr, $H_2SO_4$, $H_3PO_4$, MSA, toluene sulfonic acid and camphor sulfonic acid, preferably the acid solution is HCl.

13. The process according to any one of statements 1-12, wherein $X^1$ is halogen, preferably bromo or chloro.

14. The process according to any one of statements 1-13, wherein compound of Formula (I) is 4-bromo-2-methyl-1H-indene.

15. The process according to any one of statements 1-14, wherein $Y^1$, $Y^2$ are each independently hydroxyl or alkoxy.

16. The process according to any one of statements 1-15, wherein compound of Formula (II) is (3,5-di-t-butylphenyl) boronic acid.

17. A process for the preparation of bis(4-(3,5-di-tert-butylphenyl)-2-methyl-1H-inden-1-yl)dimethylsilane (Cpd-4),

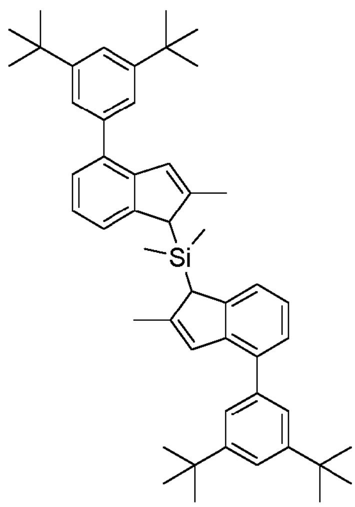

Cpd-4

the process comprising the steps of:

a) preparing 4-(3',5'-di-*t*-butylphenyl)-2-methyl-1H-indene (Cpd-3) by means of the process according to any one of statements 1 to 16; and

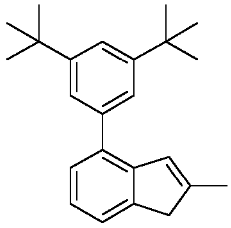
Cpd-3

b) deprotonating Cpd-3 using at least one base to form an indenide anion, followed by c) reaction with $Me_2SiX^2{}_2$, optionally in the presence of a catalyst, thereby forming bis(4-(3,5-di-tert-butylphenyl)-2-methyl-1H-inden-1-yl) dimethylsilane (Cpd-4), wherein each $X^2$ is independently selected from the group consisting of halogen, tosylate, mesylate, triflate, nonaflate, and pentafluorophenol.

18. The process according to statement 17, wherein $X^2$ is halogen, preferably chloro.

19. The process according to any one of statements 17-18, wherein the at least one base is selected from the group comprising organolithium, organomagnesium, organozinc, hydride and borane reagents, preferably the base is selected from the group comprising *n*-BuLi, *t*-BuLi, EtMgCl, LiH, $LiBEt_3H$, $NaBH_4$, $LiAlH_4$, sec-BuLi, $(nBu)_2Mg$, and MeLi, preferably n-BuLi.

20. The process according to any one of statements 17-19, wherein step (c) is performed in the presence of at least one catalyst, preferably at least one copper catalyst, preferably a copper (I) catalyst and/or copper (II) catalyst, preferably CuCN.

21. The process according to any one of statements 17-20, wherein the coupling reaction is performed in at least one solvent, preferably at least one ether-type solvent, preferably diethyl ether.

22. Process for the preparation of rac-dimethylsilylenebis(2-methyl-4-[3',5'-di-t-butylphenyl]indenyl) zirconium dichloride (*r*Met1),

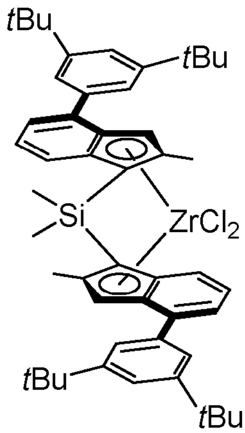
*r*Met1

the process comprising the steps of:

(a) preparing bis(4-(3,5-di-tert-butylphenyl)-2-methyl-1H-inden-1-yl)dimethylsilane (Cpd-4) by means of the process according to any one of statements 17-21; preferably step (a) comprises (a1) preparing 4-(3',5'-di-*t*-butylphenyl)-2-methyl-1H-indene (Cpd-3) by means of the process according to any one of statements 1 to 16, and (a2) deprotonating Cpd-3 using at least one base to form an indenide anion, followed by (a3) reaction with $Me_2SiX^2{}_2$, optionally in the presence of a catalyst, thereby forming bis(4-(3,5-di-tert-butylphenyl)-2-methyl-1H-inden-1-yl)dimethylsilane (Cpd-4), wherein each $X^2$ is independently selected from the group consisting of halogen, tosylate, mesylate, triflate, nonaflate, and pentafluorophenol;

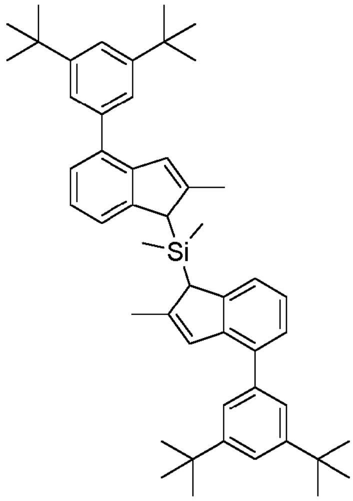

Cpd-4

(b) deprotonating Cpd-4 using at least one base to form indenide dianion, followed by (c) reaction with $ZrCl_4$, thereby forming dimethylsilylenebis(2-methyl-4-[3',5'-di-t-butylphenyl]indenyl) zirconium dichloride; and

(d) crystallizing dimethylsilylenebis(2-methyl-4-[3',5'-di-t-butylphenyl]indenyl) zirconium dichloride in toluene and isolating *rac*-dimethylsilylenebis(2-methyl-4-[3',5'-di-t-butylphenyl]indenyl) zirconium dichloride (*r*Met1).

23. The process according to statement 22, wherein the at least one base is selected from the group comprising organolithium, organomagnesium, organozinc, hydride and borane reagents, preferably the base is selected from the group comprising n-BuLi, t-BuLi, EtMgCl, LiH, $LiBEt_3H$, $NaBH_4$, $LiAlH_4$, sec-BuLi, $(nBu)_2Mg$, and MeLi, preferably n-BuLi.

24. The process according to any one of statements 22-23, wherein the coupling reaction is performed in at least one solvent, preferably at least one ether-type solvent, preferably diethyl ether.

25. Rac-dimethylsilylenebis(2-methyl-4-[3',5'-di-*t*-butylphenyl]indenyl) zirconium dichloride (*r*Met1) obtained or obtainable by a process according to any one of statements 22-24, or by a process comprising the steps of any one of the processes according to any one of statements 1-21.

26. A catalyst composition comprising:

- rac-dimethylsilylenebis(2-methyl-4-[3',5'-di-*t*-butylphenyl]indenyl) zirconium dichloride, for example as obtained according to the process according to any one of statements 22-24;

- a titanated solid oxide support, preferably a titanated silica support;

- at least one optional activator; and at least one optional co-catalyst.

27. The catalyst composition according to statement 26, comprising a titanated solid oxide support, wherein the titanated solid oxide support is a titanated solid inorganic oxide support, preferably, the solid oxide comprises silica, alumina, silica-coated alumina, aluminum phosphate, aluminophosphate, heteropolytungstate, zirconia, magnesia, boria, zinc oxide, a mixed oxide thereof, or any mixture thereof; preferably silica, silica treated with fluoride, silica-alumina, alumina treated with fluoride, sulphated alumina, silica-alumina treated with fluoride, sulphated silica-alumina, silica-coated alumina, sulfated silica-coated alumina, or any combination thereof; most preferably silica.

28. The catalyst composition according to any one of statements 26-27, comprising a titanated solid oxide support, wherein the solid oxide support has a D50 of at most 70 μm, preferably at most 60 μm, preferably at most 50 μm, preferably of at most 40 μm, preferably of at most 30 μm, preferably wherein the solid oxide support has a D50 of at least 1 μm, preferably of at least 2 μm, preferably of at least 5 μm, preferably at least 10 μm, preferably at least 15 μm, preferably at least 10 μm to at most 50, preferably at least 15 μm to at most 40 μm, preferably at least 20 μm to at most 40 μm. The D50 is defined as the particle size for which fifty percent by weight of the particles has a size lower than the

D50, as measured by laser diffraction analysis on a Malvern type analyzer.

29. The catalyst composition according to any one of statements 26-28, wherein the catalyst composition comprises at least one activator, and preferably wherein the activator comprises an aluminoxane compound, an organoboron or organoborate compound, an ionizing ionic compound, or any combination thereof, preferably wherein the activator comprises an alumoxane compound.

30. The catalyst composition according to any one of statements 26-29, wherein the catalyst composition comprises at least one activator, wherein the activator comprises at least one alumoxane compound of Formula (V) or (VI)

$R^a$-(Al($R^a$)-O)$_x$-Al$R^a_2$ (V) for oligomeric, linear alumoxanes; or
(-Al($R^a$)-O-)$_y$ (VI) for oligomeric, cyclic alumoxanes

wherein x is 1-40, and preferably 10-20;

wherein y is 3-40, and preferably 3-20; and

wherein each $R^a$ is independently selected from a $C_{1-8}$alkyl, and preferably is methyl.

31. The catalyst composition according to any one of statements 26-30, wherein the catalyst composition comprises at least one activator, wherein the activator is methyl alumoxane.

32. The catalyst composition according to any one of statements 26-31, wherein the catalyst composition comprises at least one co-catalyst.

33. The catalyst composition according to any one of statements 26-32, wherein the catalyst composition comprises at least one organoaluminum co-catalyst.

34. The catalyst composition according to any one of statements 26-33, wherein the catalyst composition comprises at least one organoaluminum co-catalyst selected from the group comprising triisobutylaluminum, trimethylaluminum, triethylaluminum, tri-n-propylaluminum, tri-n-butylaluminum, tri-n-hexylaluminum, tri-n-octylaluminum, diisobutylaluminum hydride, diethylaluminum ethoxide, diethylaluminum chloride, and any combination thereof.

35. The catalyst composition according to any one of statements 26-34, wherein the catalyst composition comprises at least 0.1 % by weight of titanium relative to the total weight of the catalyst composition as determined using ICP-AES spectroscopy, preferably at least 0.2 % by weight of titanium, preferably at least 0.3 % by weight, preferably at least 0.4 % by weight, preferably at least 0.5 % by weight of titanium, preferably at most 10.0 % by weight, preferably at most 7.0 % by weight, preferably at most 5.0 % by weight, preferably at most 3.0 % by weight, preferably at most 2.0 % by weight.

36. The catalyst composition according to any one of statements 26-35, wherein the catalyst composition comprises at least 5.0 % by weight of aluminium, relative to the total weight of the catalyst composition, preferably at least 6.0 % by weight of aluminium, preferably at least 7.0 % by weight, preferably at least 8.0 % by weight, preferably at least 9.0 % by weight, preferably at least 10.0 % by weight, for example at most 30.0 % by weight.

37. The catalyst composition according to any one of statements 26-36, wherein the catalyst composition comprises at least 700 ppm by weight of Zirconium, relative to the total weight of the catalyst composition, preferably at least 800 ppm by weight, preferably at least 900 ppm by weight, preferably at least 950 ppm by weight, preferably at most 2000 ppm by weight of Zirconium, relative to the total weight of the catalyst composition.

38. The catalyst composition according to any one of statements 26-37, comprising a titanated silica support, at least one alumoxane activator, and at least one co-catalyst.

39. A process for preparing a catalyst composition according to any one of statements 26-38, the process comprising the steps of:

- providing *rac*-dimethylsilylenebis(2-methyl-4-[3',5'-di-*t*-butylphenyl]indenyl) zirconium dichloride, preferably as prepared using the process according to any one of statements 22-25;

- contacting the rac-dimethylsilylenebis(2-methyl-4-[3',5'-di-t-butylphenyl]indenyl) zirconium dichloride with a titanated solid oxide support, thereby obtaining the catalyst composition.

40. The process according to statement 39, comprising the step of:

- contacting the rac-dimethylsilylenebis(2-methyl-4-[3',5'-di-t-butylphenyl]indenyl) zirconium dichloride with at least one cocatalyst, prior to contacting the titanated solid oxide support with the *rac*-dimethylsilylenebis(2-methyl-4-[3',5'-di-t-butylphenyl]indenyl) zirconium dichloride.

41. The process according to any one of statements 39 or 40, comprising the step of:
contacting the titanated solid oxide support with at least one activator, prior to contacting the titanated solid oxide support with the rac-dimethylsilylenebis(2-methyl-4-[3',5'-di-t-butylphenyl]indenyl) zirconium dichloride.

42. A catalyst composition obtained or obtainable by carrying out the process according to any one of statements 39-41.

43. An olefin polymerization process, the process comprising: contacting at least one catalyst composition according to any one of statements 26-38, 42, or obtained or obtainable by carrying out the process according to any one of statements 39-41, with an olefin monomer, optionally hydrogen, and optionally one or more olefin comonomers; and polymerizing the monomer, and the optionally one or more olefin comonomers, in the presence of the at least one catalyst composition, and optional hydrogen, thereby obtaining an olefin polymer.

44. The process according to statement 43, wherein the process is performed in a slurry, gas, and/or liquid phase, preferably in a slurry phase.

45. The process according to any one of statements 43 or 44, wherein the process is conducted in one or more batch reactors, slurry reactors, gas-phase reactors, solution reactors, high pressure reactors, tubular reactors, autoclave reactors, or a combination thereof.

46. The process according to any one of statements 43-45, wherein the process is conducted in a single reaction zone.

47. The process according to any one of statements 43-46, wherein the polymerization can be carried out batchwise or in a continuous process.

48. The process according to any one of statements 43-47, wherein the polymerization is carried out in a continuous process.

49. The process according to any one of statements 43-48, wherein the olefin monomer is propylene.

50. The process according to any one of statements 43-49, wherein the olefin comonomer comprises ethylene, 1-butene, 2-butene, 3-methyl-1-butene, isobutylene, 1-pentene, 2-pentene, 3-methyl-1-pentene, 4-methyl-1-pentene, 1-hexene, 2-hexene, 3-ethyl-l-hexene, 1-heptene, 2-heptene, 3-heptene, 1-octene, 1-decene, styrene, or a mixture thereof, preferably the comonomer is ethylene.

51. The process according to any one of statements 43-50, wherein the olefin monomer is propylene, and comprises the step of contacting propylene and optionally the olefin comonomer, optionally hydrogen with the catalyst composition; and obtaining a propylene polymer.

52. The process according to any one of statements 43-51, wherein the olefin monomer is propylene, and the obtained propylene polymer is a homopolymer having a melt index $MI_2$ of at least 5.0 g/10 min, preferably at least 8.0 g/10 min, preferably at most 1800 g/10 min (measured on fluff), preferably ranging from 5.0 to 300.0 g/10 min wherein $MI_2$ is determined according to ISO 1133:2005 Method B, condition M, at a temperature 230 °C, and a 2.16 kg load using a die of 2.096 mm, preferably from 5.0 g/10 min to 200.0 g/10 min, preferably from 5.0 g/10 min to 150.0 g/10 min, preferably from 8.0 g/10 min to 120.0 g/10 min.

53. The process according to any one of statements 43-52, wherein the olefin monomer is propylene, and the obtained propylene polymer is a homopolymer having a melt index $MI_{105}$ ranging from 0.30 g/10 min to 150.0 g/10 min wherein $MI_{105}$ is determined at a temperature 190 °C, and a 2.16 kg load using a die of 1.0 mm, as defined in the example

section, preferably a $MI_{105}$ ranging from 1.0 g/10 min to 140.0 g/10 min, preferably from 5.0 g/10 min to 100.0 g/10 min, preferably from 10.0 g/10 min to 100.0 g/10 min, preferably from 15.0 g/10 min to 100.0 g/10 min.

54. The process according to any one of statements 43-53, wherein the olefin monomer is propylene, and the obtained propylene polymer is a homopolymer having a xylene soluble (XS) content of at most 2.0 % by weight, preferably at most 1.5 % by weight.

55. The process according to any one of statements 43-54, wherein the olefin monomer is propylene, and the obtained propylene polymer is a homopolymer having at least one melting temperature $T_m$ of at least 150.0 °C, preferably at least 151.0 °C, preferably at least 152.0 °C, preferably at least 153.0 °C, preferably at least 154.0 °C, preferably ranging from 150.0 °C and 160.0 °C, preferably ranging from 152.0 °C and 158.0 °C, preferably ranging from 154.0 °C and 158.0 °C, with $T_m$ being defined as the melting peak at the highest temperature in °C as determined by DSC (measured on the polymer in the absence of any nucleating agent).

56. The process according to any one of statements 43-55, wherein the olefin monomer is propylene, and the obtained propylene polymer is a homopolymer having a crystallization temperature $T_c$ of at least 100.0 °C, preferably at least 105.0 °C, preferably at least 110.0 °C, preferably ranging from 105.0 °C and 130.0 °C, preferably ranging from 110.0 °C and 120.0 °C, preferably ranging from 110.0 °C and 115.0 °C, with $T_c$ as determined by DSC (measured on the polymer in the absence of any nucleating agent).

57. The process according to any one of statements 43-56, wherein the olefin monomer is propylene, and the obtained propylene polymer is a homopolymer having an amount of [2,1]-regio defects of at most 0.7 % by weight, preferably at most 0.6 % by weight, preferably at most 0.5 % by weight, as determined by the weight percentage of 2,1-insertions relative to the total weight of propylene polymer by $^{13}C$-NMR.

58. The process according to any one of statements 43-57, wherein the olefin monomer is propylene, and the obtained propylene polymer is a homopolymer having a percentage of mmmm pentads of at least 95.0 %, preferably at least 96.0 %, preferably at least 97.0 %, as determined by $^{13}C$-NMR.

59. The process according to any one of statements 43-51, wherein the olefin monomer is propylene, preferably wherein the comonomer is ethylene; and the obtained propylene polymer is a copolymer having a melt index $MI_2$ of at least 5.0 g/10 min, preferably at least 7.0 g/10 min, preferably at most 60.0 g/10 min, preferably at most 40.0 g/10 min, preferably at most 20 g/10 min, preferably ranging from 5.0 to 70.0 g/10 min, preferably ranging from 5.0 to 50.0 g/10 min, preferably ranging from 5.0 to 20.0 g/10 min, wherein $MI_2$ is determined according to ISO 1133:2005 Method B, condition M, at a temperature 230 °C, and a 2.16 kg load using a die of 2.096 mm, preferably from 5.0 g/10 min to 15.0 g/10 min, preferably from 8.0 g/10 min to 15.0 g/10 min.

60. The process according to any one of statements 43-51, 59, wherein the olefin monomer is propylene, preferably the comonomer is ethylene, and the obtained propylene polymer is a copolymer having a xylene soluble (XS) content of at most 8.0 % by weight, preferably at most 7.0 % by weight, preferably at most 6.0 % by weight, preferably at least 0.5 % by weight.

61. The process according to any one of statements 43-51, 59-60, wherein the olefin monomer is propylene, preferably the comonomer is ethylene, and the obtained propylene polymer is a copolymer having at least one melting temperature $T_m$ of at least 115.0 °C, preferably at least 120.0 °C, preferably ranging from 115.0 °C to 155.0 °C, preferably ranging from 115.0 °C to 150.0 °C, preferably ranging from 115.0 °C to 145.0 °C, preferably ranging from 115.0 °C to 140.0 °C, with $T_m$ being defined as the melting peak at the highest temperature in °C as determined by DSC (measured on the polymer in the absence of any nucleating agent).

62. The process according to any one of statements 43-51, 59-61, wherein the olefin monomer is propylene, preferably the comonomer is ethylene, and the obtained propylene polymer is a copolymer having a crystallization temperature $T_c$ of at least 80.0 °C, preferably at least 83.0 °C, preferably at least 84.0 °C, preferably ranging from 80.0 °C to 110.0 °C, preferably from 83.0 °C to 100.0 °C, with $T_c$ as determined by DSC (measured on the polymer in the absence of any nucleating agent).

63. The process according to any one of statements 43-62, wherein the olefin monomer is propylene, and the obtained propylene polymer has a molecular weight distribution $M_w/M_n$ ranging from 2.00 to 5.00, preferably from 2.00 to 4.00, preferably from 2.50 to 3.80, preferably from 2.80 to 3.50, preferably from 3.00 to 3.50, with $M_w$ being the weight-average molecular weight and $M_n$ being the number-average molecular weight.

64. The process according to any one of statements 43-63, wherein the olefin monomer is propylene, and the obtained propylene polymer has a molecular weight distribution $M_z/M_w$ ranging from 1.00 to 4.00, preferably from 1.00 to 3.00, preferably from 1.00 to 2.50, preferably from 1.50 to 2.10, preferably 1.50 to 2.00, preferably from 1.60 to 2.00, preferably from 1.70 to 2.00, with $M_z$ being the z average molecular weight and $M_w$ being the weight-average molecular weight.

65. The process according to any one of statements 43-64, wherein the olefin monomer is propylene, and the obtained propylene polymer has a molecular weight distribution $M_z/M_n$ ranging from 4.00 to 8.00, preferably from 4.50 to 7.50, preferably from 5.00 to 7.00, preferably from 5.50 to 7.00, preferably from 5.60 to 6.95, with $M_z$ being the z average molecular weight and $M_n$ being the number-average molecular weight.

66. A metallocene-catalyzed olefin polymer at least partially catalyzed by at least one catalyst composition according to any one of statements 26-38, 42, or prepared according to any one of statements 39-41, or an olefin polymer produced by the process according to any one of statements 43-65.

67. The metallocene-catalyzed olefin polymer according to statement 66, wherein said olefin polymer is a propylene polymer.

68. A metallocene-catalyzed propylene polymer at least partially catalyzed by at least one catalyst composition according to any one of statements 26-38, 42, or produced by the process according to any one of statements 39-41, or a metallocene-catalyzed propylene polymer produced by the process according to any one of statements 43-65.

69. The metallocene-catalyzed propylene polymer according to statement 68, wherein the propylene polymer is a homopolymer having a melt index $MI_2$ of at least 5.0 g/10 min, preferably at least 8.0 g/10 min, preferably at most 1800 g/10 min (measured on fluff), preferably ranging from 5.0 to 300.0 g/10 min wherein $MI_2$ is determined according to ISO 1133:2005 Method B, condition M, at a temperature 230 °C, and a 2.16 kg load using a die of 2.096 mm, preferably from 5.0 g/10 min to 200.0 g/10 min, preferably from 5.0 g/10 min to 150.0 g/10 min, preferably from 8.0 g/10 min to 120.0 g/10 min.

70. The metallocene-catalyzed propylene polymer according to any one of statements 68-69, wherein the propylene polymer is a homopolymer having a melt index $MI_{105}$ ranging from 0.30 g/10 min to 150.0 g/10 min wherein $MI_{105}$ is determined at a temperature 190 °C, and a 2.16 kg load using a die of 1.0 mm, as defined in the example section, preferably a $MI_{105}$ ranging from 1.0 g/10 min to 140.0 g/10 min, preferably from 1.0 g/10 min to 143.0 g/10 min, preferably from 5.0 g/10 min to 100.0 g/10 min, preferably from 10.0 g/10 min to 100.0 g/10 min, preferably from 15.0 g/10 min to 100.0 g/10 min.

71. The metallocene-catalyzed propylene polymer according to any one of statements 68-70, wherein the propylene polymer is a homopolymer having a xylene soluble (XS) content of at most 2.0 % by weight, preferably at most 1.5 % by weight.

72. The metallocene-catalyzed propylene polymer according to any one of statements 68-71, wherein the propylene polymer is a homopolymer having at least one melting temperature $T_m$ of at least 150.0 °C, preferably at least 151.0 °C, preferably at least 152.0 °C, preferably at least 153.0 °C, preferably at least 154.0 °C, preferably ranging from 150.0 °C and 160.0 °C, preferably ranging from 152.0 °C and 158.0 °C, preferably ranging from 154.0 °C and 158.0 °C, with $T_m$ being defined as the melting peak at the highest temperature in °C as determined by DSC (measured on the polymer in the absence of any nucleating agent).

73. The metallocene-catalyzed propylene polymer according to any one of statements 68-72, wherein the propylene polymer is a homopolymer having a crystallization temperature $T_c$ of at least 100.0 °C, preferably at least 105.0 °C, preferably at least 110.0 °C, preferably ranging from 105.0 °C and 130.0 °C, preferably ranging from 110.0 °C and 120.0 °C, preferably ranging from 110.0 °C and 115.0 °C, with $T_c$ as determined by DSC (measured on the polymer in the absence of any nucleating agent).

74. The metallocene-catalyzed propylene polymer according to any one of statements 68-73, wherein the propylene polymer is a homopolymer having an amount of [2,1]-regio defects of at most 0.7 % by weight, preferably at most 0.6 % by weight, preferably at most 0.5 % by weight, as determined by the weight percentage of 2,1-insertions relative to the

total weight of propylene polymer by $^{13}C$-NMR.

75. The metallocene-catalyzed propylene polymer according to any one of statements 68-74, wherein the propylene polymer is a homopolymer having a percentage of mmmm pentads of at least 95.0 %, preferably at least 96.0 %, preferably at least 97.0 %, as determined by $^{13}C$-NMR.

76. The metallocene-catalyzed propylene polymer according to statement 68, wherein the propylene polymer is a copolymer having a melt index $MI_2$ of at least 5.0 g/10 min, preferably at least 7.0 g/10 min, preferably at most 60.0 g/10 min, preferably, preferably at most 40.0 g/10 min, preferably at most 20 g/10 min, preferably ranging from 5.0 to 70.0 g/10 min, preferably ranging from 5.0 to 50.0 g/10 min, preferably ranging from 5.0 to 20.0 g/10 min, wherein $MI_2$ is determined according to ISO 1133:2005 Method B, condition M, at a temperature 230 °C, and a 2.16 kg load using a die of 2.096 mm, preferably from 5.0 g/10 min to 15.0 g/10 min, preferably from 8.0 g/10 min to 15.0 g/10 min, preferably wherein the comonomer is ethylene.

77. The metallocene-catalyzed propylene polymer according to any one of statements 68, 76, wherein the propylene polymer is a copolymer having a xylene soluble (XS) content of at most 8.0 % by weight, preferably at most 7.0 % by weight, preferably at most 6.0 % by weight, preferably at least 0.5 % by weight, preferably wherein the comonomer is ethylene.

78. The metallocene-catalyzed propylene polymer according to any one of statements 68, 76-77, wherein the propylene polymer is a copolymer having at least one melting temperature $T_m$ of least 115.0 °C, preferably at least 120.0 °C, preferably ranging from 115.0 °C and 155.0 °C, preferably ranging from 115.0 °C to 150.0 °C, preferably ranging from 115.0 °C to 145.0 °C, preferably ranging from 115.0 °C and 140.0 °C, with $T_m$ being defined as the melting peak at the highest temperature in °C as determined by DSC (measured on the polymer in the absence of any nucleating agent), preferably wherein the comonomer is ethylene.

79. The metallocene-catalyzed propylene polymer according to any one of statements 68, 76-77, wherein the propylene polymer is a copolymer having a crystallization temperature $T_c$ of at least 80.0 °C, preferably at least 83.0 °C, preferably at least 84.0 °C, preferably ranging from 80.0 °C to 110.0 °C, preferably from 83.0 °C to 100.0 °C, with $T_c$ as determined by DSC (measured on the polymer in the absence of any nucleating agent), preferably wherein the comonomer is ethylene.

80. The metallocene-catalyzed propylene polymer according to any one of statements 68-79, wherein the propylene polymer has a molecular weight distribution $M_w/M_n$ ranging from 2.00 to 5.00, preferably from 2.00 to 4.00, preferably from 2.50 to 3.80, preferably from 2.80 to 3.50, preferably from 3.00 to 3.50, with $M_w$ being the weight-average molecular weight and $M_n$ being the number-average molecular weight.

81. The metallocene-catalyzed propylene polymer according to any one of statements 68-80, wherein the propylene polymer has a molecular weight distribution $M_z/M_w$ ranging from 1.00 to 4.00, preferably from 1.00 to 3.00, preferably from 1.00 to 2.50, preferably from 1.50 to 2.10, preferably 1.50 to 2.00, preferably from 1.60 to 2.00, preferably from 1.70 to 2.00, with $M_z$ being the z average molecular weight and $M_w$ being the weight-average molecular weight.

82. The metallocene-catalyzed propylene polymer according to any one of statements 68-81, wherein the propylene polymer has a molecular weight distribution $M_z/M_n$ ranging from 4.00 to 8.00, preferably from 4.50 to 7.50, preferably from 5.00 to 7.00, preferably from 5.50 to 7.00, preferably from 5.60 to 6.95, with $M_z$ being the z average molecular weight and $M_n$ being the number-average molecular weight.

83. An article comprising a metallocene-catalyzed olefin polymer according to any one of statements 66-82, or comprising the polymer obtained using a process according to any one of statements 43-65.

84. The article according to statement 83, wherein the article is for melt blown applications, film applications, injections applications, blow moulding applications, rotomoulding applications, extrusion applications, and/or yarn applications.

85. The article according to any one of statements 83-84, wherein the article is selected from the group comprising fibers, blow moulded articles, caps & closures, cereal liners, blown films, yarns, rotomoulded articles, pipes, and cast films.

86. Use of a metallocene-catalyzed olefin polymer according to any one of statements 66-82, or obtained using a

process according to any one of statements 43-65, in melt blown applications, film applications, injections applications, blow moulding applications, rotomoulding applications, extrusion applications, yarn applications.

The present invention provides a process for the preparation of 4-(3',5'-di-*t*-butylphenyl)-2-methyl-1H-indene (Cpd-3),

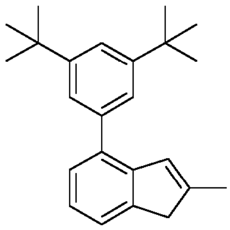

Cpd-3

the process comprising the step of:

coupling a compound of Formula (I) with a compound of Formula (II) by means of a Suzuki coupling reaction (preferably via Suzuki-Miyaura coupling reaction) thereby generating 4-(3',5'-di-*t*-butylphenyl)-2-methyl-1H-indene (Cpd-3),

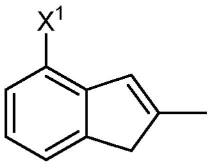

I,

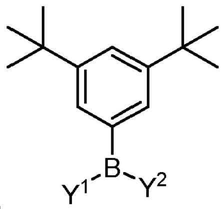

II,

wherein $X^1$ is selected from the group comprising halogen, tosylate, mesylate, triflate, nonaflate, and pentafluorophenol; and

$Y^1$, $Y^2$ are each independently selected from the group comprising hydroxyl, alkyl, alkenyl, alkynyl, and alkoxy, or $Y^1$ and $Y^2$ form together with the boron atom they are bound to a mono-, bi- or polycyclic ring.

In some embodiments, $X^1$ is bromo, fluoro, iodo or chloro. Preferably, compound of Formula (I) is 4-bromo-2-methyl-1H-indene.

In some embodiments, $Y^1$, $Y^2$ are each independently selected from the group comprising hydroxyl, $C_{1-6}$alkoxy, $C_{1-6}$alkyl, $C_{1-6}$alkenyl, and $C_{1-6}$alkynyl. In some preferred embodiments, $Y^1$, $Y^2$ are hydroxyl or $C_{1-6}$alkoxy, preferably hydroxyl. Preferably compound of Formula (II) is (3,5-di-*t*-butylphenyl)boronic acid.

The term "Suzuki coupling" as disclosed herein has a well-established meaning within the art and is intended to refer to said meaning. In particular, Suzuki coupling refers to an organic reaction where two different fragments, molecules, or compounds are joined by a carbon-carbon single bond.

Advantageously, it has been found that the coupling reaction according to the process of the present invention can proceed with high chemoselectivity, meaning that it may selectively form the desired carbon-carbon bonds without generating significant side products or competing reactions. Hence, the coupling reaction as disclosed herein may provide the target compound with minimal purification requirements.

Another advantage of the present process is that it provides a mild and step efficient synthesis of the target product without the need for excessive heating and/or long reaction times. Hence, providing a synthesis procedure which may significantly reduce operating expenses. According to the invention, coupling a compound of Formula (I) and a compound of Formula (II) is performed by means of a Suzuki coupling reaction.

In one embodiment, the coupling reaction is conducted using one or more catalysts, one or more ligands, one or more bases, and/or one or more additives. In one embodiment, the coupling reaction is conducted using one or more catalysts, one or more ligands, and one or more bases.

The metal catalyst used in the coupling reaction may contain palladium, nickel, or copper, or a combination thereof. Preferably the metal catalyst comprises palladium, preferably a palladium(II) compound.

In some embodiment, the metal catalyst is selected from the group comprising $Pd(OAc)_2$, $Pd(PPh_3)_4$, $Pd(TFA)_2$, $Pd(MeCN)_2Cl_2$, $Pd_2(dba)_3$, $PdBr_2$, or a combination of any two or more thereof, preferably $Pd(OAc)_2$.

A wide range of ligands can be used in the coupling of compound of Formula (I) and compound of Formula (II).

In some embodiments, the ligand used in the coupling reaction is an organophosphine ligand, for example an organophosphine selected from the group comprising SPhos, XPhos, APhos, CPhos, RuPhos, cataCXium, DavePhos, JohnPhos, MePhos, XantPhos, $PPh_3$, $tBuPPh_2$ and combinations thereof, preferably SPhos, XPhos, APhos, CPhos, RuPhos, cataCXium, more preferably SPhos. The structures of the listed ligand are given below.

| Phosphine | Formula | Name |
|---|---|---|
| XPhos | 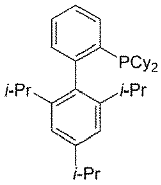 | dicyclohexyl[2',4',6'-tris(propan-2-yl)[1,1'-biphenyl]-2-yl]phosphane |
| RuPhos | 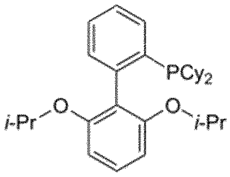 | dicyclohexyl[2',6'-di(propan-2-yloxy)[1,1'-biphenyl]-2-yl]phosphane |
| SPhos | 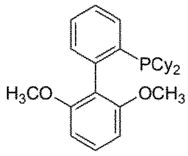 | Dicyclohexyl(2',6'-dimethoxy[1,1'-biphenyl]-2-yl)phosphane |
| DavePhos | 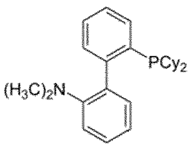 | 2-(2-dicyclohexylphosphanylphenyl)-N,N-dimethylaniline |
| JohnPhos | 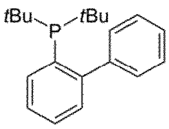 | di*tert*-butyl-(2-phenylphenyl)phosphane |
| MePhos | 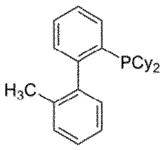 | Dicyclohexyl-[2-(2-methylphenyl)phenyl]phosphane |
| CataCXium | 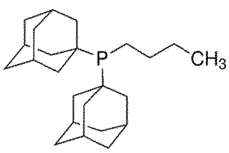 | Di(1-adamantyl)-n-butylphosphine |
| APhos | 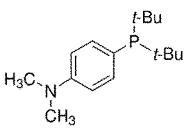 | (4-(N,N-Dimethylamino)phenyl)di-tert-butyl phosphine |
| XantPhos | 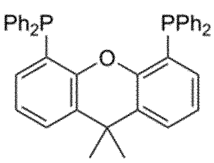 | (9,9-Dimethyl-9H-xanthene-4,5-diyl)bis(diphenylphosphane) |

(continued)

| Phosphine | Formula | Name |
|---|---|---|
| PPh3 | 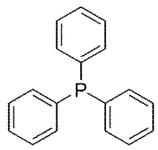 | triphenylphosphine |
| t-$BuPPh_2$ | 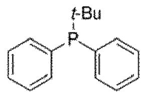 | tert-Butyldiphenylphosphine |
| $PCy_3.HBF_4$ | 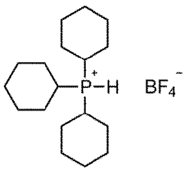 | Tricyclohexylphosphine tetrafluoroborate |
| Cy-BIPHEP | 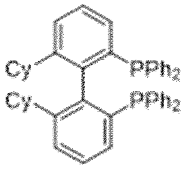 | (6,6'-dicyclohexyl-[1,1'-biphenyl]-2,2'-diyl)bis(diphenylphosphane) |

In the coupling reaction, the metal catalyst and ligand may be provided as a pre-catalyst complex, for example a Buchwald G1, G2, G3, or G4 pre-catalyst, preferably G2 or G3, complexed to a phosphine ligand, for example an organophosphine selected from the group consisting of SPhos, APhos, CPhos, RuPhos, XPhos, cataCXium, DavePhos, JohnPhos, MePhos, XantPhos, $Cy_3P-HBF_4$, Cy-BIPHEP,SPHOS-$SO_3Na$, $PPh_3$, $tBuPPh_2$ and combinations thereof. In some embodiment, the palladium precatalyst complex can be selected from the group comprising SPhos Pd G2, XPhos Pd G2, P(Cy3)Pd G3, APhos Pd G3, cataC-Pd G2 and cataC-Pd G3.

The pre-catalysts can comprise of a palladacycle (shown below) with a phenyl or 1,1-biphenyl backbone, wherein L represents a bound phosphine ligand e.g. SPhos, XPhos, etc. (see below) and wherein the bound amine substituents and the leaving group (Cl, OMs) vary. Examples of Buchwald pre-catalysts complexed with palladium and with a ligand (L) are shown below:

| G # | Pre-Catalyst complex with L | Name |
|---|---|---|
| G1 | 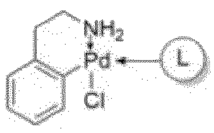 | Chloropalladium(1+);(L);2-phenylethanamine |
| G2 | 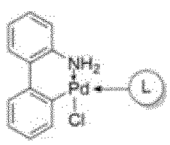 | Chloropalladium(1+);(L);2-phenylaniline |
| G3 | 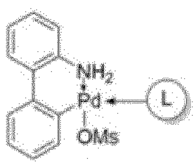 | (L);methanesulfonate;palladium(2+);2-phenylaniline |

(continued)

| G # | Pre-Catalyst complex with L | Name |
|---|---|---|
| G4 | 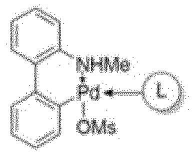 | (L);methanesulfonate;N-memthyl-2-phenylaniline;palladium(2+); |

In some embodiments, a pre-catalyst containing a phosphine ligand is used and no additional phosphine ligand is used. Alternatively, a pre-catalyst containing a phosphine ligand is used and additional phosphine ligand is also used. Examples of pre-catalysts for the coupling reaction may include $Pd(TFA)_2$, $PdBr_2$, $Pd(MeCN)_2Cl_2$, $Pd(MeCN)_2Cl_2$, $Pd(PPh_3)_4$, or $Pd_2(dba)_3$. These pre- catalysts can be used in the presence of ligands such as $Ph_2P$(t-Bu); $Cy_3P$-$HBF_4$; RuPHOS; S-PHOS, Cy-BIPHEP; or SPHOS-$SO_3$Na.

In some embodiments, the ligand used in the coupling reaction is an N-heterocyclic carbene (NHC) ligand to give complexes of the general formula $(L^x)(PR^y{}_3)(X^x)_2Ru=CHR^x$ wherein $L^x$ represents an NHC ligand such as 1,3-dimesitylimidazole-2-ylidene (IMES) and 1,3-dimesityl-4,5-dihydroimidazol-2-ylidene (sIMES), $X^x$ represents a halogen (e.g., Cl, Br, or I), $R^y$ represents an alkyl, cycloalkyl, or aryl group (e.g., butyl, cyclohexyl, or phenyl), and $R^x$ represents an alkyl, alkenyl, or aryl group (e.g., methyl, $CH=CMe_2$, phenyl, etc.). Non-limiting examples of these types of NHC ligands and catalysts are described in PCT publications WO 99/51344 and WO 00/71554, which are incorporated herein by reference.

The coupling of compound of Formula (I) and compound of Formula (II) can involve at least one base. In some embodiments, the base is an organic or inorganic salt such as $K_3PO_4$, LiOH, CsF, KF, NaF, LiF, AgF, $MgF_2$, NaOtBu, KOtBu, NaOH, KOH, MeONa, MeOK, $Cs_2CO_3$, $K_2CO_3$, $KHCO_3$, $Na_2CO_3$ and $NaHCO_3$ or a combination thereof. Preferably, the at least one base is $K_3PO_4$.

The coupling of compound of Formula (I) and compound of Formula (II) can optionally involve an additive, for example an alcohol such as ethylene glycol, for example when PdXPhos-2G/XPhos complex is used.

The coupling of compound of Formula (I) and compound of Formula (II) can be conducted in any suitable solvent. Examples of suitable organic solvents include polar solvents, non-polar solvents, protic solvents, aprotic solvents, polar protic solvents, and polar aprotic solvents. In a preferred embodiment, coupling reaction can be conducted in ether-based solvents such as THF, MeTHF, dioxane, and dialkylethers such as diethylether. Other solvents can also be used, for example halogenated alkane solvents such as dichloromethane. Alcoholic solvents, including t- amyl alcohol, hexanol, pentanol, butanol (tert-butanol, isobutanol, and n-butanol), propanol (isopropanol and n-propanol), ethanol, and/or methanol can also be used. In some preferred embodiment the solvent is selected from the group comprising THF, MeTHF, dioxane, toluene, benzene, DMF, DMSO, DMAc, IPA, MeOH and EtOH or combination thereof.

The coupling can also be conducted in an aqueous environment, including a micellar environment. Preferably, the coupling of compound of Formula (I) and compound of Formula (II) is conducted in a mixture of water and at least one organic solvent. In a preferred embodiment, a THF and water is used.

The coupling of compound of Formula (I) and compound of Formula (II) can be achieved using one or more catalysts, one or more ligands, one or more bases, and/or one or more additives. The skilled person can use their common general knowledge to determine appropriate amounts of these reagents.

In an embodiment, the catalyst or pre-catalyst is present in an amount of 0.1 mol% to 5 mol%, preferably 0.25 mol% to 3 mol%, preferably 0.5 mol% to 1.5 mol%, relative to the number of moles of compound of formula (II).

In an embodiment, the number of moles of ligand, if present, is 1 times to 3 times the number of moles of catalyst or pre-catalyst, preferably 1 to 2 times, more preferably 1.2 to 1.8 times. In an embodiment, the molar ratio of compound of formula (II) to compound of formula (I) is from 2:1 to 1:2, for example from 1.5:1 to 1:1.5, for example 1.3:1.

In an embodiment, the base is in an amount of 2 to 5 molar equivalents, preferably in an amount of 2 to 3 molar equivalents, relative to the number of moles of compound of formula (I).

In an embodiment, the catalyst is $Pd(OAc)_2$.

In an embodiment, the ligand is SPhos.

In an embodiment, compound of Formula (I) is 4-bromo-2-methyl-1H-indene.

In an embodiment, compound of Formula (II) is (3,5-di-t-butylphenyl)boronic acid.

In an embodiment, the catalyst is $Pd(OAc)_2$, the ligand is SPhos and the number of moles of SPhos is at least 1 to at most 3 times the number of moles of $Pd(OAc)_2$, preferably 1 to 2 times the number of moles of $Pd(OAc)_2$, preferably 1.4 to 2 times the number of moles of $Pd(OAc)_2$. In an embodiment, the base is $K_3PO_4$, preferably in an amount of 2 to 5 molar equivalents relative to the indene, preferably 2 to 3 molar equivalents relative to the indene.

In an embodiment, the reaction is conducted in THF and water mixture, preferably in a 1:1 ratio. In some embodiment, the reaction can be refluxed after addition of compound of formula (I).

In some embodiments, the Suzuki coupling reaction is performed over a time period of at least 30 minutes to at most 6 hours, preferably of at least 1 hour to at most 6 hours, preferably of at least 1 hour to at most 5 hours.

The present invention further encompasses 4-(3',5'-di-*t*-butylphenyl)-2-methyl-1H-indene (Cpd-3) obtained or obtainable by the process as disclosed herein.

The present invention also provides a process for the preparation of bis(4-(3,5-di-tert-butylphenyl)-2-methyl-1H-inden-1-yl)dimethylsilane (Cpd-4),

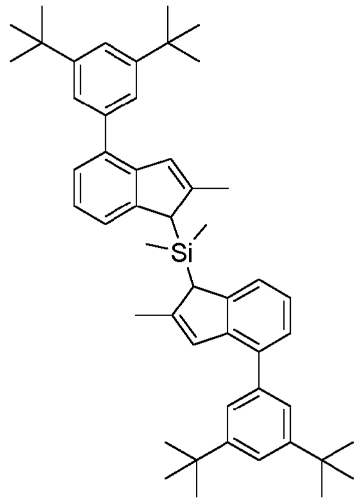

Cpd-4

the process comprising the steps of:

a) preparing 4-(3',5'-di-*t*-butylphenyl)-2-methyl-1H-indene (Cpd-3) by means of a Suzuki coupling reaction, preferably by means of a process as described herein above; and

b) deprotonating Cpd-3 using at least one base to form an indenide anion, followed by c) reaction with $Me_2SiX^2{}_2$, optionally in the presence of a catalyst, thereby forming bis(4-(3,5-di-tert-butylphenyl)-2-methyl-1H-inden-1-yl) dimethylsilane (Cpd-4), wherein each $X^2$ is independently selected from the group consisting of halogen, tosylate, mesylate, triflate, nonaflate, and pentafluorophenol.

It should be understood that a 4-(3',5'-di-*t*-butylphenyl)-2-methyl-1H-indenyl salt as disclosed herein comprises an indenyl anion (synonym indenide anion). For nomenclature purposes, the following numbering scheme is used for indenyl. It should be noted that indenyl can be considered a cyclopentadienyl with a fused benzene ring. The structure below is drawn and named as an anion:

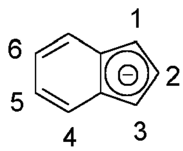

indenyl anion= indenide anion.

Preferably, the process comprises the steps of

a) coupling a compound of Formula (I) with a compound of Formula (II) by means of a Suzuki coupling reaction (preferably via Suzuki-Miyaura coupling reaction) thereby generating 4-(3',5'-di-*t*-butylphenyl)-2-methyl-1H-indene (Cpd-3), and

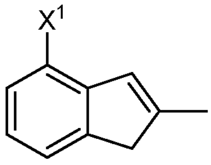 I, 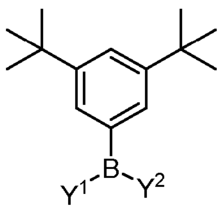 II, 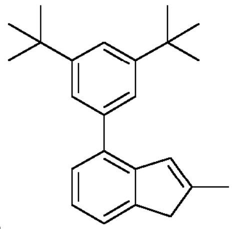 Cpd-3

b) deprotonating Cpd-3 using at least one base to form an indenide anion, followed by c) reaction with $Me_2SiX^2_2$, optionally in the presence of a catalyst, thereby forming bis(4-(3,5-di-tert-butylphenyl)-2-methyl-1H-inden-1-yl) dimethylsilane (Cpd-4),

wherein $X^1$ is selected from the group comprising halogen, tosylate, mesylate, triflate, nonaflate, and pentafluorophenol;
$Y^1$, $Y^2$ are each independently selected from the group comprising hydroxyl, alkyl, alkenyl, alkynyl, and alkoxy, or $Y^1$ and $Y^2$ form together with the boron atom they are bound to a mono-, bi- or polycyclic ring; and
each $X^2$ is independently selected from the group consisting of halogen, tosylate, mesylate, triflate, nonaflate, and pentafluorophenol.

In some embodiments, $X^2$ is halogen, preferably chloro, fluoro, bromo, or iodo; preferably $X^2$ is chloro.
The process as disclosed herein for synthesizing Cpd-4 involves deprotonating Cpd-3 with at least one base.
Non-limiting examples of suitable bases can be selected from the group comprising organolithium, organomagnesium, organozinc, hydride and borane reagents, preferably the base can be selected from the group comprising *n*-BuLi, *t*-BuLi, EtMgCl, LiH, $LiBEt_3H$, $NaBH_4$, $LiAlH_4$, sec-BuLi, $(nBu)_2Mg$, and MeLi, preferably *n*-BuLi.
In some embodiments, step (c) is performed in the presence of at least one catalyst, preferably at least one copper catalyst, preferably a copper (I) catalyst and/or copper (II) catalyst, preferably CuCN.
The reaction with $Me_2SiX^2_2$ may advantageously be performed in at least one solvent.
In preferred embodiments, the solvent comprises at least one ether-type compound. Non-limiting examples of suitable ether compounds for this purpose include diethyl ether, dimethyl ether, di-n-propyl ether, anisole, dimethoxymethane, 1,1-diethoxyethane, tetrahydrofuran, 1,4-dioxane and combinations thereof.
The present invention further encompasses bis(4-(3,5-di-tert-butylphenyl)-2-methyl-1H-inden-1-yl)dimethylsilane (Cpd-4) obtained or obtainable by the process as disclosed herein.
The present invention also provides a process for the preparation of rac-dimethylsilylenebis(2-methyl-4-[3',5'-di-t-butylphenyl]indenyl) zirconium dichloride (*r*Met1) as disclosed herein,

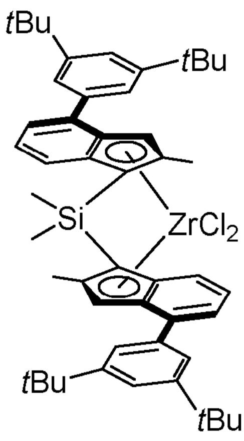 *r*Met1

the process comprising the steps of:

(i) preparing 4-(3',5'-di-*t*-butylphenyl)-2-methyl-1H-indene (Cpd-3) by means of a Suzuki coupling reaction, preferably by means of a process as described herein above;

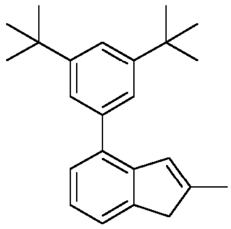

Cpd-3

(ii) preparing bis(4-(3,5-di-tert-butylphenyl)-2-methyl-1H-inden-1-yl)dimethylsilane (Cpd-4), preferably by means of a process as described herein above;

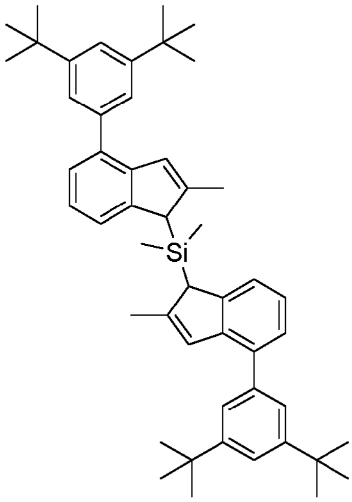

Cpd-4

(iii) deprotonating Cpd-4 using at least one base to form indenide dianion, followed by (iv) reaction with $ZrCl_4$, thereby forming dimethylsilylenebis(2-methyl-4-[3',5'-di-t-butylphenyl]indenyl) zirconium dichloride; and

(v) crystallizing dimethylsilylenebis(2-methyl-4-[3',5'-di-t-butylphenyl]indenyl) zirconium dichloride in toluene and isolating rac-dimethylsilylenebis(2-methyl-4-[3',5'-di-t-butylphenyl]indenyl) zirconium dichloride (rMet1).

Preferably, the process comprises the steps of:

a) coupling a compound of Formula (I) with a compound of Formula (II) by means of a Suzuki coupling reaction (preferably via Suzuki-Miyaura coupling reaction) thereby generating 4-(3',5'-di-*t*-butylphenyl)-2-methyl-1H-indene (Cpd-3), and

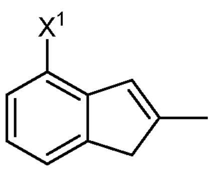 I, 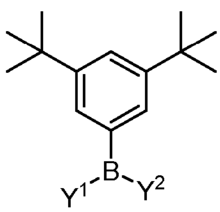 II, 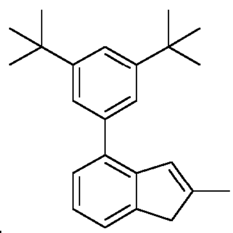 Cpd-3

b) deprotonating Cpd-3 using at least one base to form an indenide anion, followed by c) reaction with $Me_2SiX^2_2$, optionally in the presence of a catalyst, thereby forming bis(4-(3,5-di-tert-butylphenyl)-2-methyl-1H-inden-1-yl) dimethylsilane (Cpd-4),

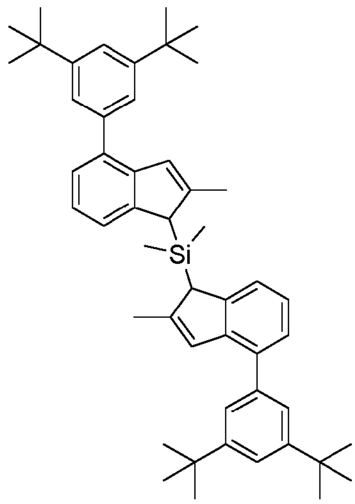

Cpd-4

(d) deprotonating Cpd-4 using at least one base to form indenide dianion, followed by (e) reaction with $ZrCl_4$, thereby forming dimethylsilylenebis(2-methyl-4-[3',5'-di-t-butylphenyl]indenyl) zirconium dichloride; and

(f) crystallizing dimethylsilylenebis(2-methyl-4-[3',5'-di-t-butylphenyl]indenyl) zirconium dichloride in toluene and isolating rac-dimethylsilylenebis(2-methyl-4-[3',5'-di-t-butylphenyl]indenyl) zirconium dichloride (*r*Met1);

wherein $X^1$ is selected from the group comprising halogen, tosylate, mesylate, triflate, nonaflate, and pentafluorophenol;
$Y^1$, $Y^2$ are each independently selected from the group comprising hydroxyl, alkyl, alkenyl, alkynyl, and alkoxy, or $Y^1$ and $Y^2$ form together with the boron atom they are bound to a mono-, bi- or polycyclic ring; and
each $X^2$ is independently selected from the group consisting of halogen, tosylate, mesylate, triflate, nonaflate, and pentafluorophenol.

The process involves deprotonating Cpd-4 using at least one base to form indenide dianion. Non-limiting examples of suitable bases include organolithium, organomagnesium, organozinc, hydride and borane reagents, preferably the base is selected from the group comprising n-BuLi, t-BuLi, EtMgCl, LiH, LiBEt3H, NaBH4, $LiAlH_4$, sec-BuLi, $(nBu)_2Mg$, and MeLi; preferably n-BuLi.

The deprotonation of Cpd-4 may advantageously be performed in at least one solvent. In preferred embodiments, the solvent comprises at least one ether-type compound. Non-limiting examples of suitable ether compounds for this purpose include diethyl ether, dimethyl ether, di-n-propyl ether, anisole, dimethoxymethane, 1,1-diethoxyethane, tetrahydrofuran, 1,4-dioxane and combinations thereof.

The next step comprises adding $ZrCl_4$, thereby forming dimethylsilylenebis(2-methyl-4-[3',5'-di-t-butylphenyl] indenyl) zirconium dichloride; crystallizing dimethylsilylenebis(2-methyl-4-[3',5'-di-t-butylphenyl]indenyl) zirconium dichloride in toluene and isolating *rac*-dimethylsilylenebis(2-methyl-4-[3',5'-di-t-butylphenyl]indenyl) zirconium dichloride (*r*Met1). The present invention further encompasses rac-dimethylsilylenebis(2-methyl-4-[3',5'-di-t-butylphenyl]indenyl) zirconium dichloride (*r*Met1) obtained or obtainable by the process as disclosed herein.

The term rac-dimethylsilylenebis(2-methyl-4-[3',5'-di-t-butylphenyl]indenyl) zirconium dichloride (*r*Met1) refers to the *rac* form of dimethylsilylenebis(2-methyl-4-[3',5'-di-t-butylphenyl]indenyl) zirconium dichloride, preferably wherein the *rac*/*meso* ratio of the rac form of dimethylsilylenebis(2-methyl-4-[3',5'-di-t-butylphenyl]indenyl) zirconium dichloride is preferably 90:10 or greater, for example 91:9 or greater, for example 92:2 or greater as determined using 1H NMR.

As used herein, the term "*rac*" "*rac* form" is used to describe a specific stereoisomer of the metallocene compound disclosed herein. The "*rac*" or "*rac* form" of the metallocene compound means that the metallocene does not have plane of symmetry containing the metal center, zirconium.

As used herein, the term "*meso*" or "*meso* form" is used to describe a specific stereoisomer of the metallocene compound disclosed herein. The *meso* or *meso* form of the metallocene compound means that the metallocene has plane of symmetry containing the metal center, zirconium.

In other words, the rac form and the *meso* form have the same molecular formula but differ in the spatial arrangement of atoms or groups in three-dimensional space.

The present invention provides a composition which comprises at least one metallocene, wherein said at least one metallocene is *rac*-dimethylsilylenebis(2-methyl-4-[3',5'-di-t-butylphenyl]indenyl) zirconium dichloride (*r*Met1), the

composition further comprising a titanated solid oxide support, preferably a titanated silica support; at least one optional activator; and at least one optional co-catalyst.

The present inventors have surprisingly found that rac-dimethylsilylenebis(2-methyl-4-[3',5'-di*t*-butylphenyl] indenyl) zirconium dichloride (*r*Met1) supported on a titanated solid oxide provides for a highly active catalyst composition, which can be used to prepare polyolefins having tailored properties.

The composition comprises at least one titanated solid oxide support. A "support", as disclosed herein, generally refers to a solid material or a solid structure, which is configured to support a catalyst. In this context, the terms "supporting", "mounting", "applying", "impregnating" are used herein as synonyms and intend to refer to the application of a catalyst on such solid support. The solid oxide support is preferably chemically unreactive with any of the components of the metallocene catalyst. In preferred embodiments, the titanated solid oxide support is a titanated solid inorganic oxide support. Suitable inorganic oxides include silica, alumina, magnesium oxide, thorium oxide, as well as mixed oxides of silica and one or more Group 2 or 13 metal oxides, such as silica-magnesia and silica-alumina mixed oxides. Silica, alumina, and mixed oxides of silica and one or more Group 2 or 13 metal oxides are preferred inorganic oxide support materials. Preferred examples of such mixed oxides are the silica-aluminas. For example the solid inorganic oxide comprises silica, alumina, silica-alumina, silica-coated alumina, aluminum phosphate, aluminophosphate, heteropolytungstate, zirconia, magnesia, boria, zinc oxide, a mixed oxide thereof, or any mixture thereof, preferably silica, silica treated with fluoride, silica-alumina, alumina treated with fluoride, sulfated alumina, silica-alumina treated with fluoride, sulfated silica-alumina, silica-coated alumina, sulfated silica-coated alumina, or any combination thereof. Most preferred support is a silica support. In a preferred embodiment, the metallocene is provided on a titanated solid inorganic oxide support, preferably a titanated silica support. The silica may be in granular, agglomerated, fumed, spray dried, or other form.

In some embodiments, the titanated solid oxide support is a porous support and preferably a porous titanated silica support having a surface area ranging from 100 to 900 $m^2/g$, preferably from 100 to 700 $m^2/g$, preferably from 100 to 500 $m^2/g$, preferably from 100 to 400 $m^2/g$, preferably from 100 to 350 $m^2/g$, preferably from 150 to 350 $m^2/g$, preferably from 200 to 350 m2/g.

In some embodiments, the titanated solid oxide support is a porous support and preferably a porous titanated silica support having an average pore volume ranging from 0.50 to 4.00 mL/g, preferably from 0.50 to 3.00 mL/g, preferably from 1.00 to 2.00 mL/g, preferably from 1.20 to 1.80 mL/g.

In some embodiments, the titanated solid oxide support, preferably the titanated silica support has a D50 of at most 70 μm, preferably of at most 50 μm, preferably of at most 40 μm. In some embodiments, the titanated solid oxide support has a D50 of at least 5 μm, preferably at least 10 μm, preferably at least 15 μm, preferably at least 20 μm, preferably at least 15 μM to at most 40 μm, preferably at least 20 μm to at most 40 μm. The D50 is defined as the particle size for which fifty percent by weight of the particles has a size lower than the D50. It is to be understood that the solid support D50 referred herein is the D50 measured for the solid support without any catalyst activator (co-catalyst).

The particle size may be measured by laser diffraction analysis on a Malvern type analyzer. The D50 is defined as the particle size for which fifty percent by weight of the particles has a size lower than the D50. The measurement of the particle size can be made according to the International Standard ISO 13320:2009 ("Particle size analysis -Laser diffraction methods"). For example, the D50 can be measured by laser diffraction analysis. For example, Malvern Instruments' laser diffraction systems may advantageously be used. The particle size may be measured by laser diffraction analysis on a Malvern type analyzer. The particle size may be measured by laser diffraction analysis on a Malvern type analyzer after having put the supported catalyst in suspension in cyclohexane. Suitable Malvern systems include the Malvern 2000, Malvern MasterSizer (such as MasterSizer S), Malvern 2600 and Malvern 3600 series. Such instruments together with their operating manual meet or even exceed the requirements set-out within the ISO 13320 Standard. The Malvern MasterSizer (such as MasterSizer S) may also be useful as it can more accurately measure the D50 towards the lower end of the range e.g., for average particle sizes of less 8 μm, by applying the theory of Mie, using appropriate optical means. In some preferred embodiments, the support has a D50 of at most 28 μm, preferably of at most 25 μm, with D50 being defined as the particle size for which fifty percent by weight of the particles has a size lower than the D50, as measured according to the International Standard ISO 13320:2009 ("Particle size analysis - Laser diffraction methods") with the MasterSizer S.

Preferably, the titanated solid oxide support may be treated with an activator, prior to supporting the metallocene compound as disclosed herein, thereby forming an activated support. The activator can be any activator known for this purpose such as an aluminum-containing activator, a boron-containing activator, or a fluorinated activator. The aluminum-containing activator may comprise an alumoxane, an alkyl aluminum, a Lewis acid and/or a fluorinated catalytic support.

In some embodiments, alumoxane is used as an activator. The alumoxane can be used in conjunction with a catalyst in order to improve the activity of the catalyst component during the polymerization reaction.

As used herein, the term "alumoxane" and "aluminoxane" are used interchangeably, and refer to a substance, which is capable of activating the bridged metallocene catalyst. In some embodiments, alumoxanes comprise oligomeric linear and/or cyclic alkyl alumoxanes. In a further embodiment, the alumoxane has formula (V) or (VI)

$R^a$-$(Al(R^a)$-$O)_x$-$AlR^a_2$ (V) for oligomeric, linear alumoxanes; or

(-$Al(R^a)$-O-$)_y$ (VI) for oligomeric, cyclic alumoxanes

wherein x is 1-40, and preferably 10-20;

wherein y is 3-40, and preferably 3-20; and

wherein each $R^a$ is independently selected from a $C_{1-8}$alkyl, and preferably is methyl. In a preferred embodiment, the alumoxane is methyl alumoxane (MAO).

In an exemplary embodiment, the catalyst composition comprises: *rac*-dimethylsilylenebis(2-methyl-4-[3',5'-di-*t*-butylphenyl]indenyl) zirconium dichloride (rMet1), a titanated silica support, and a methyl alumoxane.

The catalyst composition may further comprise a co-catalyst. One or more aluminumalkyl represented by the formula $AlR^b_x$ can be used as additional co-catalyst, wherein each $R^b$ is the same or different and is selected from halogens or from alkoxy or alkyl groups having from 1 to 12 carbon atoms and x is from 1 to 3. Non-limiting examples are Tri-Iso-Butyl Aluminum (TIBAL), Tri-Ethyl Aluminum (TEAL), Tri-Methyl Aluminum (TMA), and Methyl-Methyl-Ethyl Aluminum (MMEAL). Especially suitable are trialkylaluminums, the most preferred being triisobutylaluminum (TIBAL) and triethylaluminum (TEAL).

In a preferred embodiment, the catalyst composition comprises rac-dimethylsilylenebis(2-methyl-4-[3',5'-di-*t*-butylphenyl]indenyl) zirconium dichloride (*r*Met1), a titanated silica support, a methyl alumoxane, and at least one co-catalyst preferably selected from the group comprising TEAL, TIBAL, TMA, and MMEAL, the most preferred being TIBAL.

The present invention also provides a process for the preparation of a catalyst composition as disclosed herein, the process comprising the steps of:

- providing *rac*-dimethylsilylenebis(2-methyl-4-[3',5'-di-*t*-butylphenyl]indenyl) zirconium dichloride (*r*Met1), as disclosed herein, preferably by carrying out the process for preparing rMet1 as disclosed herein;
- contacting *r*Met1 with a titanated solid oxide support, thereby obtaining the catalyst composition.

The sequence in which the components are mixed or combined may vary. The support, *r*Met1, optional activator, and optional co-catalyst may be provided by any suitable means, e.g. by supplying said components to a suitable mixing device or vessel.

In some preferred embodiments, the process comprises contacting the titanated solid oxide support with at least one activator, such as methyl-alumoxane, prior to contacting the titanated solid oxide support with *r*Met1. Preferably the titanated solid oxide is titanated silica contacted with at least one activator.

In some embodiments, *r*Met1 is provided in a solvent prior to contacting it with the titanated solid oxide support.

In some preferred embodiments, the process comprises contacting *r*Met1 with at least one cocatalyst, prior to contacting the titanated solid oxide support with *r*Met1. Preferably, *r*Met1 is dissolved in the co-catalyst, preferably TIBAL, prior to contact with the support.

The activator-titanated solid oxide support can be suspended in a diluent, prior to contact with *r*Met1, preferably *r*Met1 and co-catalyst. Non-limiting examples of suitable diluent include hydrocarbon solvents such as aliphatic, cycloaliphatic, and aromatic hydrocarbons. Preferably, the diluent is selected from the group consisting of toluene, ethylbenzene, p-xylene, m-xylene, hexane, heptane, cyclohexane, and mixtures thereof.

In some preferred embodiments, the catalyst composition comprises at least 0.50 % by weight, preferably at least 0.75 % by weight, preferably at least 1.00 % by weight of rMet1, preferably at most 4.00 % by weight, preferably at most 2.00 % by weight, relative to the total weight of the catalyst composition.

The present invention further encompasses a catalyst composition obtained or obtainable by carrying out the process for preparing a catalyst composition as disclosed herein.

The catalyst composition as disclosed herein can be particularly useful in a process for the preparation of an olefin polymer comprising contacting at least one olefin monomer with at least one catalyst composition as disclosed herein. Preferably, said monomer is an alpha-olefin.

The present invention also encompasses an olefin polymerization process, the process comprising: contacting at least one catalyst composition according to the invention, or obtained or obtainable by carrying out the process for preparing a catalyst composition according to the invention, with at least one olefin monomer, optionally hydrogen, and optionally one or more olefin comonomers; and polymerizing the monomer, and the optionally one or more olefin comonomers, in the presence of the at least one catalyst composition, and optional hydrogen, thereby obtaining an olefin polymer.

The term "olefin" refers herein to molecules composed of carbon and hydrogen, containing at least one carbon-carbon double bond. Olefins containing one carbon-carbon double bond are denoted herein as mono-unsaturated hydrocarbons and have the chemical formula $C_nH_{2n}$, where n equals at least two. "Alpha-olefins", "$\alpha$-olefins", "1-alkenes" or "terminal olefins" are used as synonyms herein and denote olefins or alkenes having a double bond at the primary or alpha ($\alpha$) position.

Throughout the present application the terms "olefin polymer", "polyolefin" and "polyolefin polymer" may be used synonymously.

Suitable polymerization includes but is not limited to homopolymerization of an alpha-olefin, or copolymerization of the alpha-olefin and at least one other alpha-olefin comonomer.

As used herein, the term "comonomer" refers to olefin comonomers which are suitable for being polymerized with alpha-olefin monomer. The comonomer if present is different from the olefin monomer and chosen such that it is suited for copolymerization with the olefin monomer. Comonomers may comprise but are not limited to aliphatic $C_2$-$C_{20}$ alpha-olefins. Examples of suitable aliphatic $C_2$-$C_{20}$ alpha-olefins include ethylene, propylene, 1-butene, 1-pentene, 4-methyl-1-pentene, 1-hexene, 1-octene, 1-decene, 1-dodecene, 1-tetradecene, 1-hexadecene, 1-octadecene, and 1-eicosene. Further examples of suitable comonomers are vinyl acetate ($H_3C$-C(=O)O-CH=$CH_2$) or vinyl alcohol ("HO-CH=$CH_2$"). Examples of olefin copolymers suited which can be prepared can be copolymers of propylene and ethylene, copolymers of propylene and 1-butene, ethylene-hexene copolymers, ethylene-octene copolymers, copolymers of ethylene and vinyl acetate (EVA), copolymers of ethylene and vinyl alcohol (EVOH).

In some embodiments, the olefin monomer is propylene, and the olefin comonomer is selected from the group comprising ethylene, 1-butene, 2-butene, 3-methyl-1-butene, isobutylene, 1-pentene, 2-pentene, 3-methyl-1-pentene, 4-methyl-1-pentene, 1-hexene, 2-hexene, 3-ethyl-l-hexene, 1-heptene, 2-heptene, 3-heptene, 1-octene, 1-decene, styrene, and a mixture thereof. Preferably the comonomer is ethylene.

The polyolefin can be prepared out in bulk, gas, solution and/or slurry phase. The process can be conducted in one or more batch reactors, slurry reactors, gas-phase reactors, solution reactors, high pressure reactors, tubular reactors, autoclave reactors, or a combination thereof.

The polymerization can be carried out batchwise or in a continuous process. In a preferred embodiment of the present invention, the polymerization is carried out in a continuous process. The term "continuous" means a system that operates without interruption or cessation. For example, a continuous process to produce a polymer would be one where the reactants are continually introduced into one or more reactors and polymer product is continually withdrawn. By this it is meant herein that the reactors, when operating, are run in continuous mode, that is at least one feed stream is predominantly fed continuously to the reactor, while at least one stream is predominantly withdrawn continuously.

The term "slurry" or "polymerization slurry" or "polymer slurry", as used herein refers to substantially a multi-phase composition including at least polymer solids and a liquid phase, the liquid phase being the continuous phase. The solids may include the catalyst and polymerized monomer.

In some embodiments, the liquid phase comprises a diluent. As used herein, the term "diluent" refers to any organic diluent, which does not dissolve the synthesized polyolefin. As used herein, the term "diluent" refers to diluents in a liquid state, liquid at room temperature and preferably liquid under the pressure conditions in the loop reactor. Suitable diluents comprise but are not limited to hydrocarbon diluents such as aliphatic, cycloaliphatic and aromatic hydrocarbon solvents, or halogenated versions of such solvents. Preferred solvents are $C_{12}$ or lower, straight chain or branched chain, saturated hydrocarbons, $C_5$ to $C_9$ saturated alicyclic or aromatic hydrocarbons or $C_2$ to $C_6$ halogenated hydrocarbons. Non-limiting illustrative examples of solvents are butane, isobutane, pentane, hexane, heptane, cyclopentane, cyclohexane, cycloheptane, methyl cyclopentane, methyl cyclohexane, isooctane, benzene, toluene, xylene, chloroform, chlorobenzenes, tetrachloroethylene, dichloroethane and trichloroethane, preferably isobutane or hexane.

The polymerization can also be performed in gas phase, under gas phase conditions. The term "gas phase conditions" as used herein refers to temperatures and pressures suitable for polymerizing one or more gaseous phase olefins to produce polymer therefrom.

The polymerization steps can be performed over a wide temperature range. In certain embodiments, the polymerization steps may be performed at a temperature from 20 °C to 125 °C, preferably from 60 °C to 110 °C, more preferably from 60 °C to 100 °C and most preferably from 70 °C to 90 °C. Preferably, the temperature range may be within the range from 70 °C to 100 °C and most preferably from 70 °C to 90 °C. Said temperature may fall under the more general term of polymerization conditions.

In certain embodiments, in slurry conditions, the polymerization steps may be performed at a pressure from about 20 bar to about 100 bar, preferably from about 30 bar to about 50 bar, and more preferably from about 37 bar to about 45 bar. Said pressure may fall under the more general term of polymerization conditions.

The invention also encompasses a polymer at least partially catalyzed by at least one catalyst component according to the invention or at least one composition according to the invention or produced by a process according to the invention.

The present invention also encompasses a polymer, preferably an olefin polymer produced by a process as

defined herein. In some embodiments, said olefin polymer is polypropylene.

The term "propylene polymer", "polypropylene resin" or "polypropylene" as used herein refers to the propylene polymer fluff or powder that is extruded, and/or melted, and/or pelleted and can be prepared through compounding and homogenizing of the propylene polymer as taught herein, for instance, with mixing and/or extruder equipment. Unless otherwise stated, all parameters used to define the polypropylene resin, are as measured on propylene polymer pellets. The term "pellets" refers to the propylene polymer that has been pelletized, for example through melt extrusion. As used herein, the terms "extrusion" or "extrusion process", "pelletization" or "pelletizing" are used herein as synonyms and refer to the process of transforming polyolefin resin into a "polyolefin product" or into "pellets" after pelletizing. The process of pelletization preferably comprises several devices connected in series, including one or more rotating screws in an extruder, a die, and means for cutting the extruded filaments into pellets.

The term "fluff" or "powder" as used herein refers to the propylene polymer material with the solid catalyst particle at the core of each grain and is defined as the polymer material after it exits the polymerization reactor (or final polymerization reactor in the case of multiple reactors connected in series).

In an embodiment, the propylene polymer is a homopolymer. The term "propylene homopolymer" as used herein is intended to encompass polymers which consist essentially of repeat units deriving from propylene. Homopolymers may, for example, comprise at least 99.51 % by weight of repeats units derived from propylene, preferably at least 99.8%; preferably at least 99.9 % by weight of repeats units derived from propylene, as determined for example by $^{13}C$ NMR spectrometry.

The present invention also encompasses a metallocene-catalyzed propylene homopolymer, preferably prepared in the presence of at least one catalyst composition comprising: rMet1; a titanated solid oxide support, preferably a titanated silica support; an optional activator; and an optional co-catalyst;
said polymer having:
a melt index $MI_2$ of at least 5.0 g/10 min, preferably at least 8.0 g/10 min, preferably at most 1800 g/10 min (measured on fluff), preferably ranging from 5.0 to 300.0 g/10 min wherein $MI_2$ is determined according to ISO 1133:2005 Method B, condition M, at a temperature 230 °C, and a 2.16 kg load using a die of 2.096 mm, preferably from 5.0 g/10 min to 200.0 g/10 min, preferably from 5.0 g/10 min to 150.0 g/10 min, preferably from 8.0 g/10 min to 120.0 g/10 min; and/or a melt index $MI_{105}$ ranging from 0.30 g/10 min to 150.0 g/10 min wherein $MI_{105}$ is determined at a temperature 190 °C, and a 2.16 kg load using a die of 1.0 mm, as defined in the example section, preferably a $MI_{105}$ ranging from 1.0 g/10 min to 130.0 g/10 min, preferably from 5.0 g/10 min to 100.0 g/10 min, preferably from 10.0 g/10 min to 100.0 g/10 min, preferably from 15.0 g/10 min to 100.0 g/10 min.

In some embodiments, the metallocene-catalyzed propylene polymer has

a melt index $MI_2$ of at least 5.0 g/10 min, preferably at least 8.0 g/10 min, preferably at most 1800 g/10 min (measured on fluff), preferably ranging from 5.0 to 300.0 g/10 min wherein $MI_2$ is determined according to ISO 1133:2005 Method B, condition M, at a temperature 230 °C, and a 2.16 kg load using a die of 2.096 mm, preferably from 5.0 g/10 min to 200.0 g/10 min, preferably from 5.0 g/10 min to 150.0 g/10 min, preferably from 8.0 g/10 min to 120.0 g/10 min; and/or a melt index $MI_{105}$ ranging from 0.30 g/10 min to 150.0 g/10 min wherein $MI_{105}$ is determined at a temperature 190 °C, and a 2.16 kg load using a die of 1.0 mm, as defined in the example section, preferably a $MI_{105}$ ranging from 1.0 g/10 min to 130.0 g/10 min, preferably from 5.0 g/10 min to 100.0 g/10 min, preferably from 10.0 g/10 min to 100.0 g/10 min, preferably from 15.0 g/10 min to 100.0 g/10 min;

optionally a xylene soluble (XS) content of at most 2.0 % by weight, preferably at most 1.5 % by weight;

optionally at least one melting temperature $T_m$ of at least 150.0 °C, preferably at least 151.0 °C, preferably at least 152.0 °C, preferably at least 153.0 °C, preferably at least 154.0 °C, preferably ranging from 150.0 °C and 160.0 °C, preferably ranging from 152.0 °C and 158.0 °C, preferably ranging from 154.0 °C and 158.0 °C, with $T_m$ being defined as the melting peak at the highest temperature in °C as determined by DSC (measured on the polymer in the absence of any nucleating agent);

optionally a crystallization temperature $T_c$ of at least 100.0 °C, preferably at least 105.0 °C, preferably at least 110.0 °C, preferably ranging from 105.0 °C and 130.0 °C, preferably ranging from 110.0 °C and 120.0 °C, preferably ranging from 110.0 °C and 115.0 °C, with $T_c$ as determined by DSC (measured on the polymer in the absence of any nucleating agent);

optionally an amount of [2,1]-regio defects of at most 1.0 % by weight, preferably at most 0.7 % by weight, preferably at most 0.6 % by weight, preferably at most 0.5 % by weight, as determined by the weight percentage of 2,1-insertions relative to the total weight of propylene polymer by $^{13}C$-NMR;

optionally a percentage of mmmm pentads of at least 95.0 %, preferably at least 96.0 %, preferably at least 97.0 %, as determined by $^{13}C$-NMR;

optionally a molecular weight distribution $M_w/M_n$ ranging from 2.00 to 5.00, preferably from 2.00 to 4.00, preferably from 2.50 to 3.80, preferably from 2.80 to 3.50, preferably from 3.00 to 3.50, with $M_w$ being the weight-average molecular weight and $M_n$ being the number-average molecular weight;

optionally a molecular weight distribution $M_z/M_w$ ranging from 1.00 to 4.00, preferably from 1.00 to 3.00, preferably from 1.00 to 2.50, preferably from 1.50 to 2.10, preferably 1.50 to 2.00, preferably from 1.60 to 2.00, preferably from 1.70 to 2.00, with $M_z$ being the z average molecular weight and $M_w$ being the weight-average molecular weight; optionally has a molecular weight distribution $M_z/M_n$ ranging from 4.00 to 8.00, preferably from 4.50 to 7.50, preferably from 5.00 to 7.00, preferably from 5.50 to 7.00, preferably from 5.60 to 6.95, with $M_z$ being the z average molecular weight and $M_n$ being the number-average molecular weight.

In another embodiment, the polypropylene resin is a propylene copolymer. The term "propylene copolymer" as used herein is intended to encompass polymers which consist essentially of repeat units deriving from propylene and at least one other $C_2$-$C_{20}$ alpha-olefin comonomer.

As used herein, the term "comonomer" refers to olefin comonomers which are suitable for being polymerized with alpha-olefin monomer. Comonomers may comprise but are not limited to aliphatic $C_2$-$C_{20}$ alpha-olefins, preferably $C_2$-$C_{12}$ alpha-olefins. Examples of suitable aliphatic $C_2$-$C_{20}$ alpha-olefins include ethylene, 1-butene, 1-pentene, 4-methyl-1-pentene, 1-hexene, 1-octene, 1-decene, 1-dodecene, 1-tetradecene, 1-hexadecene, 1-octadecene, and 1-eicosene. In some preferred embodiments, said comonomer is ethylene.

In some embodiments, said propylene polymer is a copolymer of propylene and a higher alpha-olefin comonomer, preferably ethylene, wherein the total comonomer content, preferably ethylene (wt % C2) relative to the total weight of the propylene polymer is at least 0.5 % by weight, preferably at least 1.0 % by weight, preferably at least 1.5 % by weight, preferably at least 2.0 % by weight, as determined by $^{13}C$ NMR analysis. In some embodiments, said propylene polymer is a copolymer of propylene and a higher alpha-olefin comonomer, preferably ethylene, wherein the total comonomer content, preferably ethylene (wt % C2) relative to the total weight of the polypropylene is at most 8.0 % by weight, preferably at most 7.0 % by weight, preferably at most 6.0 % by weight, preferably at most 5.0 % by weight, vas determined by $^{13}C$ NMR analysis.

The present invention also encompasses a metallocene-catalyzed propylene copolymer, preferably prepared in the presence of at least one catalyst composition comprising: rMet1, a titanated solid oxide support, preferably a titanated silica support; an optional activator; and an optional co-catalyst; preferably wherein the comonomer is ethylene;
said polymer having:

a melt index $MI_2$ of at least 5.0 g/10 min, preferably at least 7.0 g/10 min, preferably at most 60.0 g/10 min, preferably at most 40.0 g/10 min, preferably at most 20 g/10 min, preferably ranging from 5.0 to 70.0 g/10 min, preferably ranging from 5.0 to 50.0 g/10 min, preferably ranging from 5.0 to 20.0 g/10 min, wherein $MI_2$ is determined according to ISO 1133:2005 Method B, condition M, at a temperature 230 °C, and a 2.16 kg load using a die of 2.096 mm, preferably from 5.0 g/10 min to 15.0 g/10 min, preferably from 8.0 g/10 min to 15.0 g/10 min; optionally a xylene soluble (XS) content of at most 8.0 % by weight, preferably at most 7.0 % by weight, preferably at most 6.0 % by weight, preferably at least 0.5 % by weight;

optionally at least one melting temperature $T_m$ of at least 115.0 °C, preferably at least 120.0 °C, preferably ranging from 115.0 °C and 155.0 °C, preferably ranging from 115.0 °C to 150.0 °C, preferably ranging from 115.0 °C to 145.0 °C, preferably ranging from 115.0 °C and 140.0 °C, with $T_m$ being defined as the melting peak at the highest temperature in °C as determined by DSC (measured on the polymer in the absence of any nucleating agent);

optionally a crystallization temperature $T_c$ of at least 80.0 °C, preferably at least 83.0 °C, preferably at least 84.0 °C, preferably ranging from 80.0 °C to 110.0 °C, preferably from 83.0 °C to 100.0 °C, with $T_c$ as determined by DSC (measured on the polymer in the absence of any nucleating agent);

optionally a molecular weight distribution $M_w/M_n$ ranging from 2.00 to 5.00, preferably from 2.00 to 4.00, preferably from 2.50 to 3.80, preferably from 2.80 to 3.50, preferably from 3.00 to 3.50, with $M_w$ being the weight-average molecular weight and $M_n$ being the number-average molecular weight;

optionally a molecular weight distribution $M_z/M_w$ ranging from 1.00 to 4.00, preferably from 1.00 to 3.00, preferably from 1.00 to 2.50, preferably from 1.50 to 2.10, preferably 1.50 to 2.00, preferably from 1.60 to 2.00, preferably from

1.70 to 2.00, with $M_z$ being the z average molecular weight and $M_w$ being the weight-average molecular weight;

optionally a molecular weight distribution $M_z/M_n$ ranging from 4.00 to 8.00, preferably from 4.50 to 7.50, preferably from 5.00 to 7.00, preferably from 5.50 to 7.00, preferably from 5.60 to 6.95, with $M_z$ being the z average molecular weight and $M_n$ being the number-average molecular weight.

The present invention also encompasses a process, preferably a continuous process, for the preparation of a metallocene-catalyzed propylene polymer as described herein, the process comprising: contacting a catalyst composition with propylene, optionally hydrogen, and optionally one or more alpha-olefin comonomers; and polymerizing the propylene, and the optionally one or more alpha-olefin comonomers, in the presence of the at least one catalyst composition, and optional hydrogen, thereby obtaining the metallocene-catalyzed propylene polymer as described herein.

Preferably the continuous process comprises the step of comprising: contacting at least one catalyst composition with propylene, optionally hydrogen, and optionally one or more alpha-olefin comonomers; and polymerizing the propylene, and the optionally one or more alpha-olefin comonomers, in the presence of the at least one catalyst composition, and optional hydrogen, thereby obtaining the propylene polymer as described herein,
wherein the at least one catalyst composition comprises: rMet1, a titanated solid oxide support, preferably a titanated silica support; an optional activator; and an optional co-catalyst.

The propylene polymer can be prepared in a process, preferably in a continuous process, which can be in gas, solution and/or slurry phase. The process can be conducted in one or more slurry loop reactors, gas-phase reactors, continuously stirred tank reactors or a combination thereof. Preferably the process is performed in one or more slurry loop reactor, preferably in a single slurry loop reactor.

As used herein, the terms "loop reactor" and "slurry loop reactor" may be used interchangeably herein. In certain embodiments, each loop reactor may comprise interconnected pipes, defining a reactor path. In certain embodiments, each loop reactor may comprise at least two vertical pipes, at least one upper segment of reactor piping, at least one lower segment of reactor piping, joined end to end by junctions to form a complete loop, one or more feed lines, one or more outlets, one or more cooling jackets per pipe, and one pump, thus defining a continuous flow path for a polymer slurry. The vertical sections of the pipe segments are preferably provided with cooling jackets. Polymerization heat can be extracted by means of cooling water circulating in these jackets of the reactor. The loop reactor preferably operates in a liquid full mode.

The polymerization steps can be performed over a wide temperature range. In certain embodiments, the polymerization steps may be performed at a temperature from 20 °C to 125 °C, preferably from 60 °C to 110 °C, more preferably from 60 °C to 100 °C and most preferably from 70 °C to 90 °C. Preferably, the temperature range may be within the range from 70 °C to 100 °C and most preferably from 70 °C to 90 °C. Said temperature may fall under the more general term of polymerization conditions.

The present invention also encompasses a polypropylene composition comprising the propylene polymer of the invention and one or more additives.

The additives can be for example antioxidants, UV stabilizers, pigments, processing aids, acid scavengers, lubricants, antistatic agents, fillers, nucleating agents, or clarifying agents, or combination thereof. An overview of useful additives is given in Plastics Additives Handbook, ed. H. Zweifel, 5th edition, Hanser Publishers. These additives may be present in quantities generally ranging from 0.01 to 10 % by weight based on the weight of the polypropylene composition.

After the propylene polymer is produced, it may be formed into various articles. The propylene polymer is particularly suited for articles such as fibers, melt blown or cast film products, caps and closures, cereal liners, grass yarns, rotomoulded articles, blow moulded articles, pipes, etc.

The present invention therefore also encompasses an article comprising a propylene polymer as defined herein; or obtained according to a process as defined herein. In some embodiments, said article can be fibers, film products such as melt blown or cast film products, caps and closures, rotomoulded article, pipes, blow moulded articles, preferably melt blown moulded articles, etc. Preferred embodiments for propylene polymer of the invention are also preferred embodiments for the article of the invention.

The invention also encompasses a process for preparing an article according to the invention. Preferred embodiments as described above are also preferred embodiments for the present process.

The following examples serve to merely illustrate the invention and should not be construed as limiting its scope in any way. While the invention has been shown in only some of its forms, it should be apparent to those skilled in the art that it is not so limited, but is susceptible to various changes and modifications without departing from the scope of the invention.

## Examples

TEST METHODS

The properties cited herein and cited below were determined in accordance with the following test procedures. Where any of these properties is referenced in the appended claims, it is to be measured in accordance with the specified test procedure.

**Melt flow index**

The melt flow index ($MI_2$) of polypropylene was determined according to ISO 1133:2005 Method B, condition M, at a temperature 230 °C, and a 2.16 kg load using a die of 2.096 mm. $MI_{105}$ was used to measure melt indexes $MI_2$ higher than 300 g/10 min. $MI_{105}$ was determined at a temperature 190 °C, and a 2.16 kg load using a die of 1.0 mm.

Correlation between the above two MI measures was determined by measuring the melt flow index of several polypropylene grades using both methods. The results are shown in Table A. Polypropylene Lumicene® MR 2002 and Polypropylene Lumicene® MH140 are metallocene-catalyzed propylene homopolymers commercially available from Total Energies. Lumicene® MR30MC2, and Lumicene® MR60MC2 are metallocene-catalyzed random copolymer polypropylenes commercially available from TotalEnergies. PPR-Ref is a reference metallocene-catalyzed random propylene copolymer (synthesis described below). All samples were powders (fluff as obtained at the exit of the reactor), 1 % by weight of BHT (2,6-tertbutyl-4-methylphenol) was added before the analysis.

Table A

| Grade | $MI_2$ meas. (g/10 min) | $MI_{105}$ meas. (g/10 min) |
|---|---|---|
| MR2002 | 17.9 | 0.96 |
| MH140 | 150.5 | 9.21 |
| MH140 | 146.3 | 9.25 |
| PPR-Ref | 11.0 | 0.66 |
| MR30MC2 | 31.0 | 1.87 |
| MR60MC2 | 67.7 | 4.09 |

A linear correlation was found, with a coefficient of 16.57 between $MI_{105}$ and $MI_2$ as shown in Figure 1. The linear regression gave a very good correlation factor ($r^2$ = 0,999) and the slope corresponds to the ratio $MI_2/MI_{105}$. The calculated coefficient was used to calculate very low melt index.

PPR-Ref was prepared using *rac*-methyl(cyclohexyl)silanediyl-bis[(2-methyl-4-(4-tert-butylphenyl)indenyl]zirconium dichloride (CAS No: 888227-55-2) supported on silica which has been previously activated with methylalumoxane (MAO). The polymerization reaction was conducted in a double-loop reactor, (two loop reactors connected in series), pre-polymerization was performed in a smaller loop reactor, connected to the first loop reactor of the double loop reactor. The polymerization conditions and results are given in Table B.

Table B

| Polymer | | | PPR-ref |
|---|---|---|---|
| Pre-polymerization | ALKYL | type | TeAl |
| | ALKYL | ppm | 10 |
| | Temperature | °C | 14 |
| | Propylene | kg/h | 50 |
| | Hydrogen | Nl/h | 0 |
| | Ethylene | kg/h | 0 |
| | Residence time | min | 5.5 |

(continued)

| Polymer | | | PPR-ref |
|---|---|---|---|
| First loop reactor | Temperature | °C | 70 |
| | Propylene | kg/h | 42 |
| | Ethylene | kg/h | 1.8 |
| | Hydrogen | Nl/h | 25 |
| | Contribution | wt.% | 59.1 |
| | $H_2/C_3$ | | 0.27 |
| | Residence time | min | 48 |
| | $MI_2$ | g/10min | 10 |
| Second loop reactor | Temperature | °C | 70 |
| | Propylene | kg/h | 45 |
| | Ethylene | kg/h | 1.0 |
| | Hydrogen | Nl/h | 11.0 |
| | Contribution | wt.% | 40.9 |
| | $H_2/C_3$ | | 0.24 |
| | Residence time | min | 33 |
| | $MI_2$ | g/10min | 11.0 |
| | C2 | wt.% | 2 |

**Molecular weight, molecular distribution**

The molecular weight ($M_n$ (number average molecular weight), $M_w$ (weight average molecular weight), $M_z$ (z-average molecular weight)) and molecular weight distributions D ($M_w/M_n$), D' ($M_z/M_w$), and $M_z/M_n$ were determined by size exclusion chromatography (SEC) and in particular by gel permeation chromatography (GPC). Briefly, a GPC-IR5 from Polymer Char was used: 10 mg polymer sample was dissolved at 160 °C in 10 mL of trichlorobenzene (technical grade) for 1 hour. Injection volume: about 400 μL, automatic sample preparation and injection temperature: 160 °C. Column temperature: 145 °C. Detector temperature: 160 °C. Two Shodex AT-806MS (Showa Denko) and one Styragel HT6E (Waters) columns were used with a flow rate of 1 mL/min. Mobile Phase: trichlorobenzene stabilized with 1000 ppm by weight of butylhydroxytoluene (BHT) filtered through a 0.45 μm PTFE filter. Detector: Infrared detector (2800-3000 $cm^{-1}$) to measure the concentration, one narrow filter center based at 2928 $cm^{-1}$, and one narrow filter center based at 2959 $cm^{-1}$. Calibration: narrow standards of polystyrene (PS) (commercially available).

Calculation of molecular weight $M_i$ of each fraction i of eluted polymer is based on the Mark-Houwink relation ($\log_{10}(M_{PP}) = \log_{10}(M_{PS}) - 0.25323$ (cut off on the low molecular weight end at $M_{PP}$ = 1000).

The molecular weight averages used in establishing molecular weight/property relationships are the number average ($M_n$), weight average ($M_w$) and z average ($M_z$) molecular weight. These averages are defined by the following expressions and are determined form the calculated $M_i$:

$$M_n = \frac{\sum_i N_i M_i}{\sum_i N_i} = \frac{\sum_i W_i}{\sum_i W_i / M_i} = \frac{\sum_i h_i}{\sum_i h_i / M_i}$$

$$M_w = \frac{\sum_i N_i M_i^2}{\sum_i N_i M_i} = \frac{\sum_i W_i M_i}{\sum_i W_i} = \frac{\sum_i h_i M_i}{\sum_i h_i}$$

$$M_z = \frac{\sum_i N_i M_i^3}{\sum_i N_i M_i^2} = \frac{\sum_i W_i M_i^2}{\sum_i W_i M_i} = \frac{\sum_i h_i M_i^2}{\sum_i h_i M_i}$$

Here $N_i$ and $W_i$ are the number and weight, respectively, of molecules having molecular weight $M_i$. The third mathematical expression in each equation presented above (farthest right) defines how one obtains these averages from SEC chromatograms. hi is the height (from baseline) of the SEC curve at the $i_{th}$ elution fraction and $M_i$ is the molecular weight of species eluting at this increment.

## Xylene soluble content (XS content)

Xylene soluble content was determined by dissolving the propylene polymer in refluxing xylene, cooling the solution to 25 °C, filtering the solution, and subsequently evaporating the solvent. The residue, which is the xylene soluble portion of the propylene polymer, was then dried and weighed. The protocol was as follows: around 7.5 g of propylene polymer (PP) was weighed into a flask and 250 mL xylene were added. The xylene was heated under stirring and then left to reflux for 15 minutes, until dissolution. Heating and stirring were stopped, and the solution was left standing for 15 minutes exactly. The flask was then placed in a thermostated bath set to 25 °C +/- 1 °C for 1 hour. The solution was filtered through Whatman n° 4 filter paper and exactly 50 ml of solution were collected. The solution was then evaporated and the residue dried and weighed. The percentage of xylene solubles ("XS") was then calculated according to:
XS (in wt.%) = (Weight of the residue / Initial total weight of PP) * 500 with all weights being in the same units.

## Differential Scanning Calorimetry (DSC) for Determination of Crystallization and Melting Temperatures.

Peak crystallization temperature ($T_c$), peak melting temperature ($T_m$), crystallization enthalpy ($\Delta H_c$), and melting enthalpy ($\Delta H_m$) were measured via Differential Scanning using DQ 2000 instrument by TA Instruments, calibrated with indium and using T zero mode. The polymer analysis was performed with a 2 to 5 mg of polymer sample. The sample was first equilibrated at 40 °C and subsequently heated to 250 °C using a heating rate of 20 °C/min (first heat). The sample was held at 250 °C for 3 min to erase any prior thermal and crystallization history. The sample was subsequently cooled down to 0 °C with a constant cooling rate of 20 °C/min (first cool). The sample was held isothermal at 0 °C for 2 min before being heated to 350 °C at a constant heating rate of 20 °C/min (second heat). The exothermic peak of crystallization (first cool) was analyzed using the TA Universal Analysis software and the peak crystallization temperature ($T_c$) corresponding to 20 °C/min cooling rate was determined. The endothermic peak of melting (second heat) was also analyzed using the TA Universal Analysis software and the peak melting temperature ($T_m$) corresponding to 20 °C/min heating rate was determined. Unless otherwise indicated, reported values of $T_c$, $T_m$ in this invention refer to a cooling and heating rate of 20 °C/min, respectively.

## $^{13}$C NMR/ Tacticity

Tacticity was determined by $^{13}$C-NMR spectroscopy. The $^{13}$C-NMR spectroscopic analysis was performed at an operative frequency of 125 MHz using a 500 MHz Bruker NMR spectrometer with a high temperature 10 mm cryoprobe under conditions such that the signal intensity in the spectrum is directly proportional to the total number of contributing carbon atoms in the sample. Such conditions are well known to the skilled person and include for example appropriate relaxation time, etc. In practice, the intensity of a signal is obtained from its integral, i.e. the corresponding area. The data were acquired using proton decoupling, 240 scans per spectrum, a pulse repetition delay of 11 s and a spectral width of 26000 Hz at a temperature of 130 °C. The sample was prepared by dissolving a sufficient amount of polymer in 1,2,4-trichlorobenzene (TCB, 99 %, spectroscopic grade) at 130 °C and occasional agitation to homogenize the sample, followed by the addition of hexadeuterobenzene ($C_6D_6$, spectroscopic grade) and a minor amount of hexamethyldisiloxane (HMDS, 99.5+ %), with HMDS serving as internal standard. To give an example, about 600 mg of polymer was dissolved in 2.0 mL of TCB, followed by addition of 0.5 mL of $C_6D_6$ and 2 to 3 drops of HMDS.

Following data acquisition, the chemical shifts are referenced to the signal of the internal standard HMDS, which is assigned a value of 2.03 ppm.

The isotacticity is determined by $^{13}$C-NMR spectroscopic analysis on the total polymer according to procedures well known in the art. In the spectral region of the methyl groups the signals corresponding to the pentads *mmmm, mmmr, mmrr* and *mrrm* are assigned using published data, for example A. Razavi, Macromol. Symp., vol. 89, pages 345-367. Only the pentads *mmmm, mmmr, mmrr* and *mrrm* are taken into consideration. The other remaining pentads are neglected due to the weak intensity of the corresponding signals. For the signal relating to the mmrr pentad a correction is performed for its overlap with a methyl signal related to 2,1-insertions. The percentage of *mmmm* pentads is then calculated according to

$$\%\ mmmm = AREA\,mmmm / (AREA\,mmmm + AREA\,mmmr + AREA\,mmrr + AREA\,mrrm) * 100$$

The regio-defects content in the polypropylene is the percentage of 2,1-insertions in the polypropylene.

For propylene homopolymers, the signals corresponding to the 2,1-insertions are identified with the aid of published data, for example H.N. Cheng, J. Ewen, Makromol. Chem., vol. 190 (1989), pages 1931 -1940.

For propylene copolymers, the determination of the percentage of 2,1-insertions is detailed hereunder with respect to ethylene as comonomer but can be applied with other comonomers. The determination of the percentage of 2,1-insertions for a copolymer of propylene with ethylene as comonomer is determined by two contributions:

(i) the percentage of 2,1-insertions as defined above for the propylene homopolymer, and
(ii) the percentage of 2,1-insertions, wherein the 2,1-inserted propylene neighbors an ethylene, Thus, the total percentage of 2,1-insertions corresponds to the sum of these two contributions. A first area, AREA1, is defined as the average area of the signals corresponding to 2,1-insertions. A second area, AREA2, is defined as the average area of the signals corresponding to 1,2-insertions. The assignment of the signals relating to the 1,2-insertions is well known to the skilled person. The percentage of 2,1-insertions is calculated according to

$$2,1\text{-insertions (in \%)} = AREA1 / (AREA1 + AREA2) * 100$$

with the percentage in 2,1-insertions being given as the molar percentage of 2,1-inserted propylene with respect to total propylene.

The assignments of the signal for case (ii) can be done either by using reference spectra or by referring to the published literature.

**Comonomer content**

Comonomer content of the polypropylene: The total comonomer content (in particular ethylene ($C_2$) i.e. $C_2$ % by weight) relative to the total weight of the polypropylene composition was determined using $^{13}C$ NMR.

The sample was prepared by dissolving a sufficient amount of polymer in 1,2,4-trichlorobenzene (TCB 99 % spectroscopic grade) at 130 °C and occasional agitation to homogenize the sample, followed by the addition of hexadeuterobenzene ($C_6D_6$, spectroscopic grade) and a minor amount of hexamethyldisiloxane (HMDS, 99.5+ %), with HMDS serving as internal standard. To give an example, about 600 mg of polymer was dissolved in 2.0 mL of TCB, followed by addition of 0.5 mL of $C_6D_6$ and 2 to 3 drops of HMDS.

$^{13}C$ NMR signal was recorded on a Bruker 500 MHz with a 10 mm probe using the conditions listed in the Table C.

Table C

| |
|---|
| Pulse angle : 90° |
| Pulse repetition time : 20 s |
| Spectral width : 25000 Hz centered at 95 ppm |
| Data points : 64 K |
| Temperature : 130 °C +/-2 °C |
| Rotation : 15 Hz |
| Scan numbers : 2000 - 4000 |
| Decoupling sequence : inverse-gated decoupling sequence to avoid NOE effect |

$^{13}C\{^1H\}$ NMR spectrum was obtained by Fourier Transform on 131 K points after a light Gaussian multiplication. Spectrum was phased, baseline corrected and chemical shift scale was referenced to the internal standard HMDS at 2.03 ppm.

Chemical shifts of signals are peak picked and peaks are integrated as shown in Figure 2 and in the following Table D.

Table D

| Region | Shift (ppm) | Region | Shift (ppm) | Region | Shift (ppm) |
|---|---|---|---|---|---|
| A | 22.80-19.6 | I | 27.74-27.54 | W | 30.12-29.84 |
| B | 42.37-42.18 | P | 34.92-34.73 | X | 27.55-27.10 |

(continued)

| Region | Shift (ppm) | Region | Shift (ppm) | Region | Shift (ppm) |
|---|---|---|---|---|---|
| C | 38.64-38.43 | Q | 34.67-34.50 | Y | 24.94-24.26 |
| D | 36.04-35.81 | R | 34.06-33.9 | | |
| E | 31.6-31.49 | S | 31.4-31.21 | | |
| F | 30.63-30.51 | T | 38.34-37.7 | | |
| G | 17.71-17.49 | U | 37.7-37.37 | | |
| H | 17.31-17.10 | V | 30.46-30.31 | | |

Chemical shits are given at +/- 0.05 ppm.

The main peak A is indicative of 1, 2 PP units.

Peak I is indicative of 1.3 PP units.

Peaks B, C, D, E, F, G, H are indicative of 2,1 PP units.

Peaks T, U, V, W, X, Y are indicative of ethylene incorporation in 1,2 PP unit

Peaks P, Q, R, S are indicative of ethylene incorporation after 2.1 PP unit

The % by weight of the $C_2$ content is obtained by the following areas (A) combination

$$A_{C3\ 1,2} = A_A$$

$$A_{C3\ 2,1} = (A_B + A_C + A_D + A_E + A_F + A_G + A_H)/7 + (0.5*A_P + 0.5*A_Q + 0.5*A_S)/3$$

$$A_{C3\ 1,3} = A_I / 2$$

$$A_{C2\ E1\ 1,2} = (0.5*A_T + A_Y)/2$$

$$A_{C2\ E2\ 1,2} = (A_U - A_I + A_X)/2 - A_V$$

$$A_{C2\ E3\ 1,2} = ((A_U - A_I + A_T)*0.5 + (A_X - A_V)*0.5 + A_V + A_W + A_X + A_Y)/2 - A_{C2\ E1\ 1,2} - A_{C2\ E2\ 1,2}$$

$$A_{C2\ 2,1} = (0.5*A_P + 0.5*A_Q + 0.5*A_S + A_R)/4$$

$$A_{C2} = (A_{C2\ E1\ 1,2} + A_{C2\ E2\ 1,2} + A_{C2\ E3\ 1,2} + A_{C2\ 2,1})$$

$$A_{C3} = (A_{C3\ 1,2} + A_{C3\ 2,1} + A_{C3\ 1,3})$$

$$\%(wt.)\ C2 = (28 * A_{C2}) / (28 * A_{C2} + 42 * A_{C3}) \times 100$$

$$Randomness\ (\%) = A_{C2\ E1\ 1,2} / A_{C2}$$

**Test specimen**

The polypropylene resins were injected on a Netstal 60 injection moulding machine to produce injection molded ISO bars in accordance with ISO 294-1:1996

**Flexural modulus**

The flexural modulus was determined according to ISO 178:2010 method A with the conditions listed in Table E.

Table E

| | |
|---|---|
| Temperature | (T° 23 °C ± 2 °C) |
| Test machine | Zwick flexural test machine Z005 associated with KUKA robot |
| Force sensor | 500 N cell |
| Displacement transducer | Extensometer Zwick type BT2-EXCOMFL.001 |
| Norm | ISO 178: 2010 method A |
| Test specimen | bar 80 mm x 10 mm x 4 mm cut from type 1A specimen (ISO 294-1:1996) |
| Pre-charge | 0.5 N |
| Test speed | 2 mm/min |
| Span between specimen supports | 64 mm |
| End of the test | The module was measured between 0.05 % et 0.25% deformation |
| Relative humidity | 50 % ±10 % |

**Mechanical properties**

The tensile modulus was determined at 23 °C according to ISO 527-1:2012 using an extensometer (Method B) on injection moulded specimens type 1A, with 4 mm sample thickness. The test speed was 50 mm/min. Elastic modulus is obtained during the tensile strength test. It is the slope of the line between 0.05 and 0.25 % deformation.

Resilience was determined using Notched Izod Impact Strength measured according to ISO 180/A1: 2006 at 23 °C, V notch sample type 1A, with the conditions listed in Table F.

Table F

| | |
|---|---|
| Temperature | 23 °C |
| Impact energy | 2.75 J |
| Impact velocity | 3.5 m/s |
| Notch type | V-Notch Type 1A |
| initial angle | 124° |
| Test specimen | bar 80 mm x 10 mm x 4 mm cut from type 1A specimen |

**Racemic/ Meso Ratio of metallocene catalyst**

The racemic / meso ratio of the *racemic* form of dimethylsilylenebis(2-methyl-4-[3',5'-di-t-butylphenyl]indenyl) zirconium dichloride (*r*Met1) was determined from $^1H$ NMR spectrum. The sample was prepared by dissolving a few dozen mg of solid complex in 0.5 mL of anhydrous methylene chloride ($CD_2Cl_2$, spectroscopic grade) at room temperature. $^1H$ NMR signal was recorded on a Bruker 400 MHz with a 5 mm probe with the following conditions:

Pulse angle: 90°

Pulse repetition time: 60s

Spectral width: 6000 Hz centered at 5.5 ppm

Data points: 32K

Temperature: 25 °C +/- 1 °C

Rotation: 20 Hz

Scan numbers: 8

$^1$H NMR spectrum was obtained by Fourier Transform on 32K points after a light exponential multiplication. Spectrum was phased, baseline corrected, and chemical shift scale was referenced to the $CH_2Cl_2$ peak at 5.29 ppm.

Chemical shifts of signals were peak picked, and peaks were integrated as mentioned on Figure 4 and in the following Table G.

Table G

| | Peak position | High Integral limit | Low Integral limit |
|---|---|---|---|
| | (ppm) | (ppm) | (ppm) |
| Racemic | 2.24 | 2.42 | 1.92 |
| Meso | 2.44 | 2.47 | 2.42 |

The rac/meso ratio was obtained by the following areas (A) combination:

$$Rac / Meso = A_{Racemic} / A_{Meso}$$

<u>**Determination of Al, Zr, and Ti contents of the supported catalyst**</u>

The Al, Zr and Ti contents were determined using inductively coupled plasma atomic emission spectroscopy (ICP-AES) after acid digestion of the catalyst. The spectrometer used was ICP-AES ARCOS, by Spectro.

The determination of the elements was carried out by nebulization of the solution in an argon plasma, measurement of the intensities of the most sensitive and interference-free emission lines and comparison of these intensities with those of calibration solutions (external calibration method).

<u>Preparation of the solution to be analyzed (test solution):</u> Under an inert atmosphere (in a glove box), about 0.3 g of catalyst were added into a platinum crucible and 3 to 5 mL of isopropyl alcohol were added to "deactivate" the catalyst. The mixture was heated to dryness in a sand bath (30 minutes). The platinum crucible was placed in an oven at 600 °C for 10 minutes. After cooling, Milli-Q® deionized water was added to impregnate all the ashes, and 1 mL of concentrated HCl (Merck HCl 32% v/v) and concentrated HF (Merck HF 48% v/v) were added. The crucible was placed in a sand bath, and Milli-Q® deionized water was added to mix the content of the crucible. After 24 h, 1 mL of concentrated HCl, 0.5 mL of concentrated HF and Milli-Q® deionized water were added while agitating the mixture under heat to achieve full dissolution. After cooling the mixture was transferred to a 50 mL polypropylene tube and the volume made up to 50 mL with Milli-Q® deionized water. The test solutions were then diluted from 10 to 100 times ensuring that 2% HCl/HF1% medium was maintained.

<u>Preparation of calibration standards and control solutions:</u> Standard solutions were prepared by dilution of commercial single-element solutions of certified concentrations. The standard solutions were prepared by transferring the required volume of the certified solution to a 50 mL polypropylene tube, then rinsing the sides of the tube with Milli-Q® deionized water, and adding 1 mL of concentrated HCl and 0.5 mL of concentrated HF per 50 mL to obtain the same acid content in solution as in the sample solutions, and finalizing the dilution with Milli-Q® deionized water. Control solutions were prepared by dilution of commercial multi-element solutions of certified concentrations. The presence of other elements in solution allowed verification of the presence/absence of possible interferences.

**Determination of Al, Zr, and Ti contents in the polymers**

The Al, Zr and Ti content was determined using inductively coupled plasma atomic emission spectroscopy (ICP-AES) after mineralization of the sample and recovery of the residues in an acid medium. The spectrometer used was ICP-AES ARCOS, by Spectro.

The determination of the elements was carried out by nebulization of the solution in an argon plasma, measurement of the intensities of the most sensitive and interference-free emission lines and comparison of these intensities with those of calibration solutions (external calibration method).

Preparation of the solution to be analyzed (test solution): About 10 g of sample were added into a platinum crucible and put into an automatic furnace for calcination. The furnace has stages up to 600°C in order to keep combustion under control. After cooling, Milli-Q® deionized water was added to impregnate all the ashes, and 1mL of concentrated HCl (Merck HCl 32% v/v) and concentrated HF (Merck HF 48% v/v) were added. The crucible was placed in a sand bath, and Milli-Q® deionized water was added to mix the content of the crucible. After 24 h, 1 mL of concentrated HCl, and Milli-Q® deionized water were added while agitating the mixture under heat to achieve full dissolution. After cooling the mixture was transferred to a 50 mL polypropylene tube and the volume made up to 50 mL with Milli-Q® deionized water.

Preparation of calibration standards and control solutions: Standard solutions were prepared by dilution of commercial single-element solutions of certified concentrations. The standard solutions were prepared by transferring the required volume of the certified solution to a 50 mL polypropylene tube, then rinsing the sides of the tube with Milli-Q® deionized water, and adding 1 mL of concentrated HCl per 50 mL to obtain the same acid content in solution as in the sample solutions, and finalizing the dilution with Milli-Q® deionized water. Control solutions were prepared by dilution of commercial multi-element solutions of certified concentrations. The preparation protocol is the same as for the standards solutions. The presence of other elements in solution allowed verification of the presence/absence of possible interferences.

Expression of results for Zr, Al and Ti contents:

The content (in ppm) of the element measured in the sample was calculated as follows:

$$\frac{\text{Concentration in mg/L of the element in solution x Volume (50 mL) x Dilution factor}}{Mass\ (g)}$$

The Limit of Quantification (LOQ) was calculated for each element from 10 blank measurements:

$$\text{LOQ in solution (mg/l) = standard deviation of 10 replicates of the blank x 10}$$

$$\text{LOQ in sample (ppm)} = \frac{\text{LQ in solution x Volume (50 mL) x Dilution factor}}{Mass\ (g)}$$

or the limit of quantifications are experimentally determined usings external controls solutions.

**Particle size, D50**

The particle size, D50 of the catalyst support was determined according to the International Standard ISO 13320:2009 ("Particle size analysis -Laser diffraction methods").

**Catalyst synthesis**

Abbreviations used in the description, particularly in the Schemes and Examples, are as follows: AcOH: Acetic acid; aq.: Aqueous; DCM: Dichloromethane; TIBAL: triisobutylaluminum; DIBAL: Diisobutylaluminum hydride; DMF: N,N-Dimethylformamide; DMSO: Dimethylsulfoxide; Equiv: Equivalent; $Et_2O$: Diethyl ether; EtOAc: Ethyl acetate; EtOH: Ethanol; h: Hour(s); HPLC: High performance liquid chromatography; ILG: Leaving group; ACN: Acetonitrile; MeOH: Methanol; min: Minute; NMR: Nuclear Magnetic Resonance; $Pd(OAc)_2$: Palladium(II) acetate; rt: Room temperature; Rt: Retention time; RM: Reaction mixture; TeAl: Tri-Ethyl Aluminum; THF: Tetrahydrofuran; SPhos: (Dicyclohexyl(2',6'-dimethoxy[1,1'-biphenyl]-2-yl)phosphane); UV: Ultraviolet.

All starting materials which are not explicitly described were either commercially available (the details of suppliers such as for example TCI, Fluorochem , Acros Organics, Sigma-Aldrich, ThermoFisher, Combi-Blocks, Enamine, MatrixScientific, Merck, Chempure, Angene, BLDpharm, Apollo Scientific, Nanjing, Avra, etc. can be found in the SciFinder® Database for example) or the synthesis thereof has already been described precisely in the specialist literature (experimental guidelines can be found in the Reaxys® Database or the SciFinder® Database respectively, for example) or can be prepared using the conventional methods known to the person skilled in the art. All solvent used herein were degassed.

***Synthesis of Rac*-dimethylsilylenebis(2-methyl-4-[3',5'-di-t-butylphenyl]indenyl) zirconium dichloride (*r*Met1)**

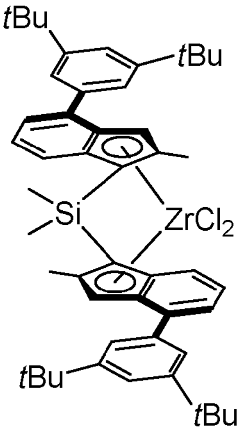

***r*Met1**

Rac-dimethylsilylenebis(2-methyl-4-[3',5'-di-t-butylphenyl]indenyl) zirconium dichloride (***r*Met1**) was prepared as described below. Unless otherwise mentioned, all procedures take place in a glovebox under a nitrogen atmosphere using dry solvents.

**1. Synthesis of 4-(3',5'-di-t-butylphenyl)-2-methyl-1H-indene (Cpd-3)**

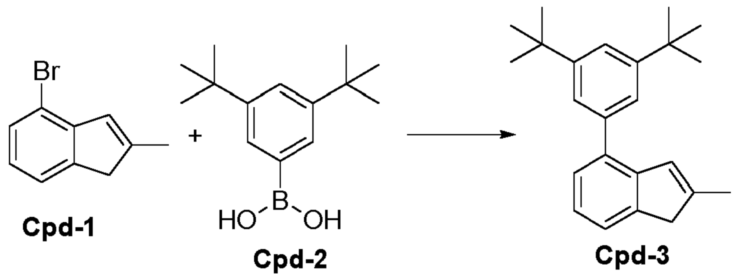

**Cpd-1** **Cpd-2** **Cpd-3**

$Pd(OAc)_2$ (0.027 g, 0.120 mmol) and Dicyclohexyl(2',6'-dimethoxy[1,1'-biphenyl]-2-yl)phosphane (SPhos) (0.074 g, 0.179 mmol) were combined in a flask with 10 mL of dry THF, thereby forming a catalytic mixture.

In a 250 mL flask (3,5-di-tert-butylphenyl)boronic acid (**Cpd-2**) (3.64 g, 15.54 mmol) and $K_3PO_4$ (6.73 g, 31.69 mmol) were added and the flask was evacuated with $N_2$ / vacuum. To this flask, a degassed mix 1:1 of $H_2O$ / THF was added and then 2.5 g (11.96 mmol) of 4-bromo-2-methyl-1H-indene (**Cpd-1**) was dissolved therein. The catalytic mixture was then added. The flask was fitted with a condenser and the reaction mixture was refluxed for 2h. Full conversion was obtained once the catalytic mixture turned black.

HCl (1M, 80 mL) was used to quench the reaction and the reaction mixture was extracted three times with 25 mL of EtOAc. The combined organic phases were dried over $MgSO_4$ and evaporated.

The product was dissolved in heptane and then purified through a silica column. The column was washed 3 times with 30 mL of heptane.

The heptane fractions were collected and evaporated under reduced pressure to yield 3.8 g of an oily residue. The oil was crystallized after 24h into **Cpd-3.**

$^1H$ NMR (400 MHz, $CDCl_3$): δ 1.32 (18H, s), 1.62 (3H, s), 3.37 (2H, d, *J* = 11.5 Hz), 6.51 (1H, s), 7.10 (1H, dd, *J* = 8.2, 1.6 Hz), 7.27-7.47 (2H, 7.32 (t, *J* = 1.3 Hz), 7.40 (dd, *J* = 8.5, 8.2 Hz), 7.62-7.80 (3H, 7.67 (dd, *J* = 1.6, 1.3 Hz), 7.73 (dd, *J* = 8.5, 1.6 Hz).

**2. Synthesis of bis(4-(3,5-di-tert-butylphenyl)-2-methyl-1H-inden-1-yl)dimethylsilane (Cpd-4)**

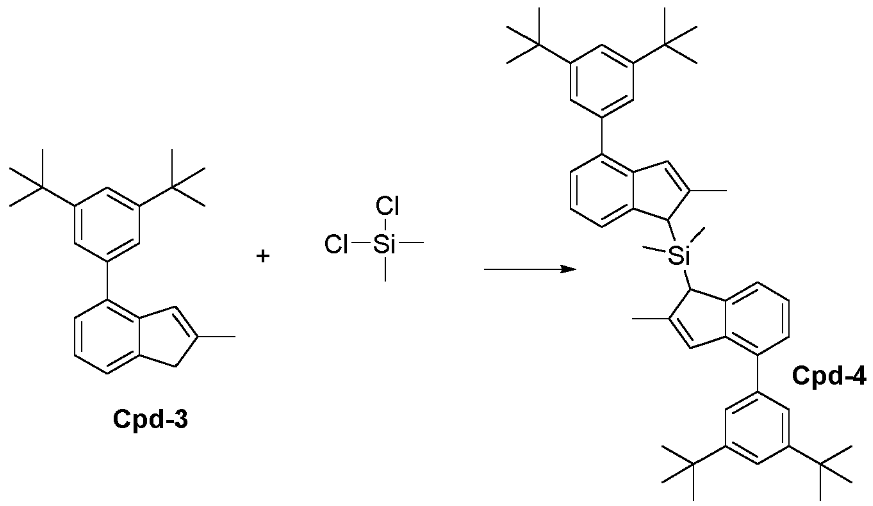

Cpd-3

Cpd-4

Deprotonation: n-BuLi (1.6 M, 0.841g, 8.203 ml, 13.124 mmol) was added dropwise to a solution of **Cpd-3** (3.800 g, 11.931 mmol) in 50 mL of diethyl ether and left to react overnight at room temperature. CuCN (0.045 g, 0.501 mmol) was added to the reaction mixture, followed by $SiMe_2Cl_2$ (0.862 g, 6.681 mmol) 15 minutes later. A clear solid formed in suspension. The mixture was left to stir for 24 hours at room temperature. The then solvent was evaporated and DCM was added. The suspension was filtered on silica using DCM as solvent giving a clear yellow filtrate.

After evaporation of the DCM, the obtained oil was dissolved in 50mL of n-pentane. MeOH 50 mL was then added. The sonication of this mixture gave a milky oil. The mixture was then concentrated *in vacuo* giving an oil.

50 mL of MeOH were added to the oil, and subsequently evaporated under vacuum. Another 50 mL of MeOH were added and the solid obtained was crushed to form a white suspension. The mixture was left to stir in MeOH overnight which led to the formation of a clear yellow methanol phase and a white powder, which was filtered over a glass filter thereby yielding **Cpd-4.** Yield: 2.2 g, 57%. $^1H$ NMR (400 MHz, $CDCl_3$) is shown in Figure 3.

**3. Synthesis of rac-dimethylsilylenebis(2-methyl-4-[3',5'-di-t-butylphenyl]indenyl) zirconium dichloride (rMet1)**

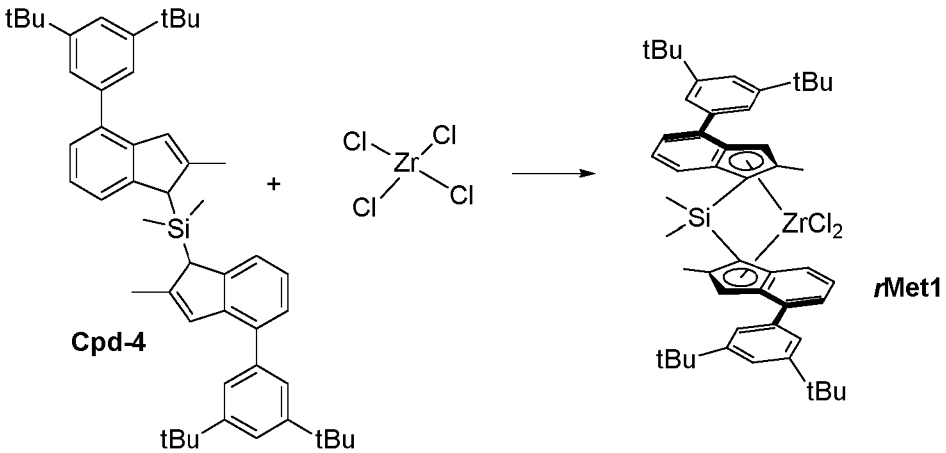

Cpd-4

***r*Met1**

**Cpd-4** (2.2 g, 3.3 mmol) was dissolved in 50 mL of $Et_2O$ and n-BuLi (1.6 M, 0.508 g, 4.96ml, 7.935 mmol) was added dropwise at room temperature. The mixture was left to stir overnight (orange color) at room temperature. $ZrCl_4$ (0.814g, 3.491 mmol) was added at room temperature and the mixture was left to stir for a day.

The solvent was evaporated, and the residue obtained was dissolved in toluene, and filtered through celite. The filtrate was left to crystallize to yield ***r*Met1** (200mg, yield: 10%). The *rac* amount of the crystal was determined by $^1H$ NMR (Figure 4). Based on these results, it was concluded that ***r*Met1** at the end of the crystallization step had rac purity higher than 93% (*rac/meso* molar ratio 19.5).

**Preparation of the titanated silica:**

Synthetic amorphous silica ES757 type CS8136 from Ecovyst was used as catalyst support. The properties of the silica are shown in Table 1.

Table 1

| Property | Values |
|---|---|
| Surface Area ($m^2/g$) | 292 |
| Pore Volume (mL/g) | 1.47 |
| Malvern D20 (μm) | 15.4 |
| Malvern D50 (μm) | 26.7 |
| Malvern D90 (μm) | 46.2 |

with D90 being defined as the particle size for which ninety percent by weight of the particles has a size lower than the D90;

with D20 being defined as the particle size for which twenty percent by weight of the particles has a size lower than the D20;

with D50 being defined as the particle size for which fifty percent by weight of the particles has a size lower than the D50; and

with the D90, D10 and D50 being measured by laser diffraction analysis on a Malvern type analyzer.

The silica support (5kg) was introduced in an activator vessel incorporating a fluidized bed, flushed under nitrogen and the temperature was raised from 50 °C to 270 °C (1°C/min). The dehydration step was then carried out at this temperature for 3 hours. After the dehydration step, TYZOR® TPT (a mixture of 80 wt% Ti isopropoxide and 20 wt% tertiary butoxide titane), stored under anhydrous nitrogen, was progressively injected in the fluidized bed (over 60 min whilst maintaining the temperature at 270 °C). The amount of titanium tetraisopropoxide injected was calculated in order to give the required titanium content in the resultant catalyst (for 1% Ti = 64 ml Tyzor/Kg silica). The obtained titanated silica was heated up to a temperature of at least 450 °C (1 °C/min) and maintained at this temperature for 6 hours. The titanated silica was cooled down to 50 °C (10 °C/min) and maintained at 50 °C under nitrogen until unloading. For unloading, the titanated silica was left to cool under nitrogen and was unloaded and kept under inert atmosphere prior to further use.

**Preparation of the supported catalyst composition:**

1 kg of dried titanated silica (prepared as described above) was suspended in toluene (20 wt. % solid suspension) and the suspension was stirred at 100 rpm. 2.2 kg of methyl-alumoxane (MAO) (30 % by weight in toluene) was added dropwise at 20°C and the resulting suspension was heated to 110 °C (reflux) for 4 hours under mild stirring. The suspension was cooled to room temperature and the mixture was then filtered. The recovered solids were washed 3 times with both dry toluene (20-25 wt. % solid suspension) and then dried under reduced pressure overnight yielding the MAO-support.

The metallocene rMet1 (loading target 1.25 wt. % of supported metallocene) was dissolved for one hour at 20 °C in TIBAL (0.15 Kg of 10 wt.% TIBAL solution per gram of rMet1). After onehour, the MAO-support was suspended in toluene at 20 °C and slowly added to the metallocene/TIBAL mixture. The mixture was stirred at room temperature for 2 hours at 20 °C and then filtered. The solid was washed 3 times with isohexane (20-25 wt. % solid suspension), filtered and then dried at room temperature under reduced pressure. The supported catalyst was slurried in mineral oil (Finavestan A360 from TotalEnergies) in order to have a solid content of about 20 wt. %.and stored at 0-5°C.

The catalyst composition was analyzed for titanium, zirconium and aluminum content (wt.%) using ICP-AES spectroscopy (Inductively Coupled Plasma - Atomic Emission Spectroscopy). The results are shown in Table 2.

Table 2

| Catalyst composition | Al (wt.%) | Zr (ppm) | Ti (wt.%) |
|---|---|---|---|
| *r*Met1/Ti-silica/MAO/TIBAL | 12.0 | 1000 | 0.9 |

EXAMPLE A - Homo-polymerization of propylene:

The polymerization reaction was conducted in a single loop reactor. Pre-polymerization was performed in a smaller loop reactor, connected to the loop reactor. The polymerization conditions and analytical results for the obtained polymers are shown in Table 3.

Normal liter (NL) refers to the volume occupied by a gas delivered under normal temperature and pressure conditions, i.e. under a Temperature (T) of 298 K and pressure (p) of 101325 Pa.

Table 3: operating conditions and analytical results for propylene homopolymer.

| Polymers | | | A | B | C | D |
|---|---|---|---|---|---|---|
| Pre-polymerization | ALKYL: TeAl | ppm | 14 | 14 | 14 | 14 |
| | Temperature | °C | 15.0 | 15.0 | 15.0 | 15.0 |
| | Propylene | kg/h | 39 | 39 | 39 | 39 |
| | Hydrogen | Nl/h | 0 | 0 | 0 | 0 |
| | Ethylene | kg/h | 0 | 0 | 0 | 0 |
| | Residence time | min | 6.6 | 6.6 | 6.6 | 6.6 |
| Loop reactor Operating conditions | Temperature | °C | 72.0 | 72.0 | 72.0 | 72.0 |
| | Propylene | Kg/h | 45.0 | 45.0 | 45.0 | 45.0 |
| | Hydrogen | Nl/h | 89.0 | 39.0 | 31.0 | 22.0 |
| | $H_2/C_3$- feed | NL/kg | 1.06 | 0.46 | 0.37 | 0.26 |
| | Residence time | min | 50 | 50 | 50 | 50 |
| Analytical Results* | $MI_2$ | g/10min | 1430* | 101.0 | 34.0 | 10.8 |
| | $MI_{105}$ | g/10min | 86.3 | | | |
| | Bulk density (BD) | g/cm$^3$ | 0.35 | 0.37 | 0.40 | 0.41 |
| | XS content | wt.% | 1.3 | 0.8 | 0.7 | 0.7 |
| GPC | $M_n$ | Da | 15749 | 35117 | 49466 | 67054 |
| | $M_w$ | Da | 53931 | 109662 | 152262 | 208358 |
| | $M_2$ | Da | 101946 | 205343 | 290406 | 406502 |
| | $M_w/M_n$ | | 3.42 | 3.12 | 3.08 | 3.11 |
| | $M_z/M_w$ | | 1.89 | 1.87 | 1.91 | 1.95 |
| | $M_z/M_n$ | | 6.47 | 5.85 | 5.87 | 6.06 |
| DSC | Peak $T_m$ | °C | 154.9 | 155.5 | 155.6 | 155.8 |
| | Melting enthalpy | J/g | 92.0 | 94.7 | 87.6 | 91.1 |
| | Melting onset | °C | 151.9 | 152.8 | 152.8 | 152.7 |
| | Peak $T_c$ | °C | 112.3 | 111.9 | 111.1 | 111.6 |
| | Crystal enthalpy | J/g | 97.1 | 100.6 | 94.3 | 96.7 |
| NMR | 2,1 insertions | wt.% | 0.4 | 0.4 | 0.4 | 0.4 |
| | mmmm pentads | % | 97.6 | 98.5 | 98.4 | 98.9 |
| *Analytical results were measured on the pellets, except for Polymer A which was measured on the fluff. NL = Normal liter.* | | | | | | |

GPC trace of each of the polymers A to D are shown in Figures 5-8 respectively.

It can be seen that the catalyst composition displays good hydrogen response. The produced polymers have a high melting point whatever the melt index range.

The produced polymers showed a combination of high %mmmm (≥97.6 %) and low defect (≤0.4%). Low XS

content were also observed.

EXAMPLE B - Copolymerization of propylene:

The polymerization reaction was conducted in a single loop reactor. Pre-polymerization was performed in a smaller loop reactor, connected to the loop reactor. The polymerization conditions and analytical results for the polymers obtained are shown in Table 4.

Table 4: operating conditions and analytical results for propylene copolymer.

| **Polymers** | | | **E** | **F** | **G** |
|---|---|---|---|---|---|
| Pre-polymerization | ALKYL: TeAl | ppm | 14 | 14 | 14 |
| | Temperature | °C | 15.0 | 15.0 | 15.0 |
| | Propylene | kg/h | 39 | 39 | 39 |
| | Hydrogen | Nl/h | 0 | 0 | 0 |
| | Ethylene | kg/h | 0 | 0 | 0 |
| | Residence time | min | 6.6 | 6.6 | 6.6 |
| Loop reactor Operating conditions | Temperature | °C | 70.0 | 70.0 | 70.0 |
| | Propylene | Kg/h | 45.0 | 45.0 | 45.0 |
| | Hydrogen | Nl/h | 13.2 | 16.0 | 17.0 |
| | $H_2$/C3- feed | NL/kg | 0.16 | 0.19 | 0.20 |
| | Ethylene | Kg/h | 1.85 | 3.10 | 2.55 |
| | C2 Off gas | (wt.%) | 1.88 | 2.80 | 2.47 |
| | Residence time | min | 48 | 48 | 48 |
| Analytical Results | $MI_2$ | g/10min | 9.9 | 11.2 | 13.4 |
| | Bulk density (BD) | g/cm$^3$ | 0.46 | 0.25 | 0.40 |
| | XS content | wt.% | 1.1 | 5.1 | 2.9 |
| GPC | $M_n$ | Da | 65696 | 57192 | 58671 |
| | $M_w$ | Da | 204639 | 197199 | 182505 |
| | $M_z$ | Da | 402288 | 392146 | 355207 |
| | $M_w/M_n$ | | 3.11 | 3.45 | 3.11 |
| | $M_z/M_w$ | | 1.97 | 1.99 | 1.95 |
| | $M_z/M_n$ | | 6.12 | 6.86 | 6.05 |
| DSC | Peak $T_m$ | °C | 136.4 | 123.3 | 128.7 |
| | Melting enthalpy | J/g | 69.7 | 56.3 | 65.0 |
| | Melting onset | °C | 131.7 | 116.9 | 123.4 |
| | Peak $T_c$ | °C | 96.8 | 85.4 | 90.3 |
| | Crystal enthalpy | J/g | 71.8 | 57.7 | 68.1 |
| NMR | C2 | wt.% | 2.3 | 4.0 | 3.3 |
| *NL = Normal liter.* | | | | | |

GPC trace of each of the polymers E to F are shown in Figures 9-11 respectively.
NMR analysis revealed that C2 (ethylene) incorporation was high.
Mechanical properties were analyzed and the results are shown in Table 5.

Table 5

| Polymers | | | E | G |
|---|---|---|---|---|
| Mechanical properties | Tensile modulus | MPa | 1019 ± 9 | 855 ± 31 |
| | Flexural modulus | MPa | 1045 ± 13 | 864 ± 18 |
| | Resilience at 23°C | $kJ/m^2$ | 3.34 ± 0.05 | 3.66 ± 0.20 |

Resilient materials with good mechanical properties could be obtained.

## Claims

1. A process for the preparation of 4-(3',5'-di-*t*-butylphenyl)-2-methyl-1H-indene (Cpd-3),

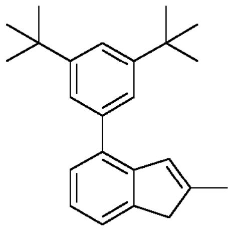

Cpd-3

the process comprising the step of:

coupling a compound of Formula (I) with a compound of Formula (II) by means of a Suzuki coupling reaction (preferably via Suzuki-Miyaura coupling reaction) thereby generating 4-(3',5'-di-*t*-butylphenyl)-2-methyl-1H-indene (Cpd-3),

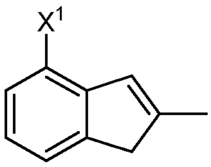

I,

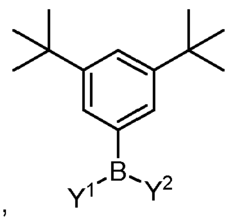

II,

wherein $X^1$ is selected from the group comprising halogen, tosylate, mesylate, triflate, nonaflate, and pentafluorophenol; and
$Y^1$, $Y^2$ are each independently selected from the group comprising hydroxyl, alkyl, alkenyl, alkynyl, and alkoxy, or $Y^1$ and $Y^2$ form together with the boron atom they are bound to a mono-, bi- or polycyclic ring.

2. The process according to claim 1, wherein said coupling is performed in the presence of at least one metal catalyst, wherein the metal catalyst comprises palladium, nickel, or copper, or a combination thereof, preferably said metal catalyst is a palladium catalyst.

3. The process according to any one of claims 1-2, wherein said coupling is performed in the presence of at least one palladium catalyst and at least one organophosphine ligand, or in the presence of at least one palladium precatalyst complex.

4. The process according to any one of claims 1-3, wherein said coupling is performed in the presence of at least one base.

5. The process according to any one of claims 1-4, wherein said coupling is performed in at least one solvent, preferably in at least one organic solvent, for example in a mixture of water and at least one organic solvent.

6. The process according to any one of claims 1-5, wherein compound of Formula (I) is 4-bromo-2-methyl-1H-indene.

**7.** The process according to any one of claims 1-6, wherein compound of Formula (II) is (3,5-di-t-butylphenyl)boronic acid.

**8.** A process for the preparation of bis(4-(3,5-di-tert-butylphenyl)-2-methyl-1H-inden-1-yl)dimethylsilane (Cpd-4),

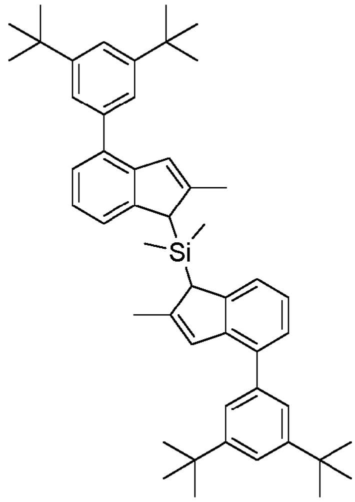

Cpd-4

the process comprising the steps of:

a) preparing 4-(3',5'-di-*t*-butylphenyl)-2-methyl-1H-indene (Cpd-3) by means of the process according to any one of claims 1 to 7; and

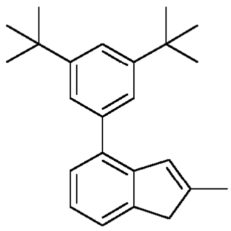

Cpd-3

b) deprotonating Cpd-3 using at least one base to form an indenide anion, followed by c) reaction with $Me_2SiX^2{}_2$, optionally in the presence of a catalyst, thereby forming bis(4-(3,5-di-tert-butylphenyl)-2-methyl-1H-inden-1-yl) dimethylsilane (Cpd-4), wherein each $X^2$ is independently selected from the group consisting of halogen, tosylate, mesylate, triflate, nonaflate, and pentafluorophenol.

**9.** Process for the preparation of *rac*-dimethylsilylenebis(2-methyl-4-[3',5'-di-t-butylphenyl]indenyl) zirconium dichloride (rMet1),

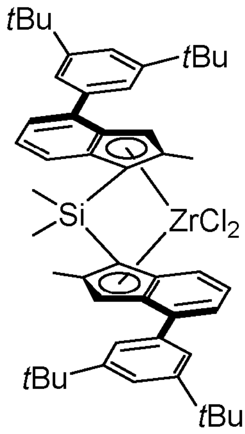

rMet1

the process comprising the steps of:

(a) preparing bis(4-(3,5-di-tert-butylphenyl)-2-methyl-1H-inden-1-yl)dimethylsilane (Cpd-4) by means of the process according to claim 8;

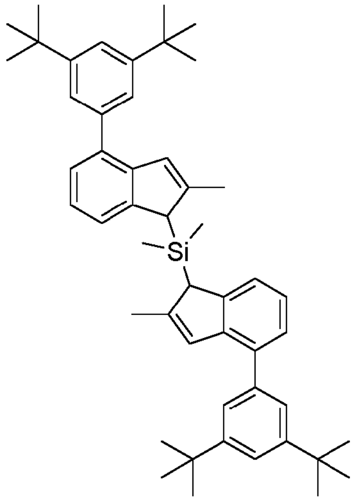

Cpd-4

(b) deprotonating Cpd-4 using at least one base to form indenide dianion, followed by (c) reaction with $ZrCl_4$, thereby forming dimethylsilylenebis(2-methyl-4-[3',5'-di-t-butylphenyl]indenyl) zirconium dichloride; and
(d) crystallizing dimethylsilylenebis(2-methyl-4-[3',5'-di-t-butylphenyl]indenyl) zirconium dichloride in toluene and isolating rac-dimethylsilylenebis(2-methyl-4-[3',5'-di-t-butylphenyl]indenyl) zirconium dichloride (rMet1).

**10.** A catalyst composition comprising:

- rac-dimethylsilylenebis(2-methyl-4-[3',5'-di-*t*-butylphenyl]indenyl) zirconium dichloride, for example as obtained according to the process according to claim 9;
- a titanated solid oxide support, preferably a titanated silica support;
- at least one optional activator; and at least one optional co-catalyst.

**11.** The catalyst composition according to claim 10, comprising rac-dimethylsilylenebis(2-methyl-4-[3',5'-di-*t*-butylphenyl]indenyl) zirconium dichloride, a titanated silica support, at least one alumoxane activator, and at least one co-catalyst.

**12.** A process for preparing a catalyst composition according to any one of claims 10-11, the process comprising the steps of:

- providing *rac*-dimethylsilylenebis(2-methyl-4-[3',5'-di-*t*-butylphenyl]indenyl) zirconium dichloride, preferably as

prepared using the process according to claim 9;
- contacting the *rac*-dimethylsilylenebis(2-methyl-4-[3',5'-di-*t*-butylphenyl]indenyl) zirconium dichloride with a titanated solid oxide support, thereby obtaining the catalyst composition.

**13.** The process according to claim 12, comprising the step of:
contacting the *rac*-dimethylsilylenebis(2-methyl-4-[3',5'-di-t-butylphenyl]indenyl) zirconium dichloride with at least one cocatalyst, prior to contacting the titanated solid oxide support with the *rac*-dimethylsilylenebis(2-methyl-4-[3',5'-di-t-butylphenyl]indenyl) zirconium dichloride.

**14.** The process according to any one of claims 12 or 13, comprising the step of:
contacting the titanated solid oxide support with at least one activator, prior to contacting the titanated solid oxide support with the *rac*-dimethylsilylenebis(2-methyl-4-[3',5'-di-t-butylphenyl]indenyl) zirconium dichloride.

**15.** An olefin polymerization process, the process comprising: contacting at least one catalyst composition according to any one of claims 10-11, or obtained or obtainable by carrying out the process according to any one of claims 12-14, with an olefin monomer, optionally hydrogen, and optionally one or more olefin comonomers; and polymerizing the monomer, and the optionally one or more olefin comonomers, in the presence of the at least one catalyst composition, and optional hydrogen, thereby obtaining an olefin polymer.

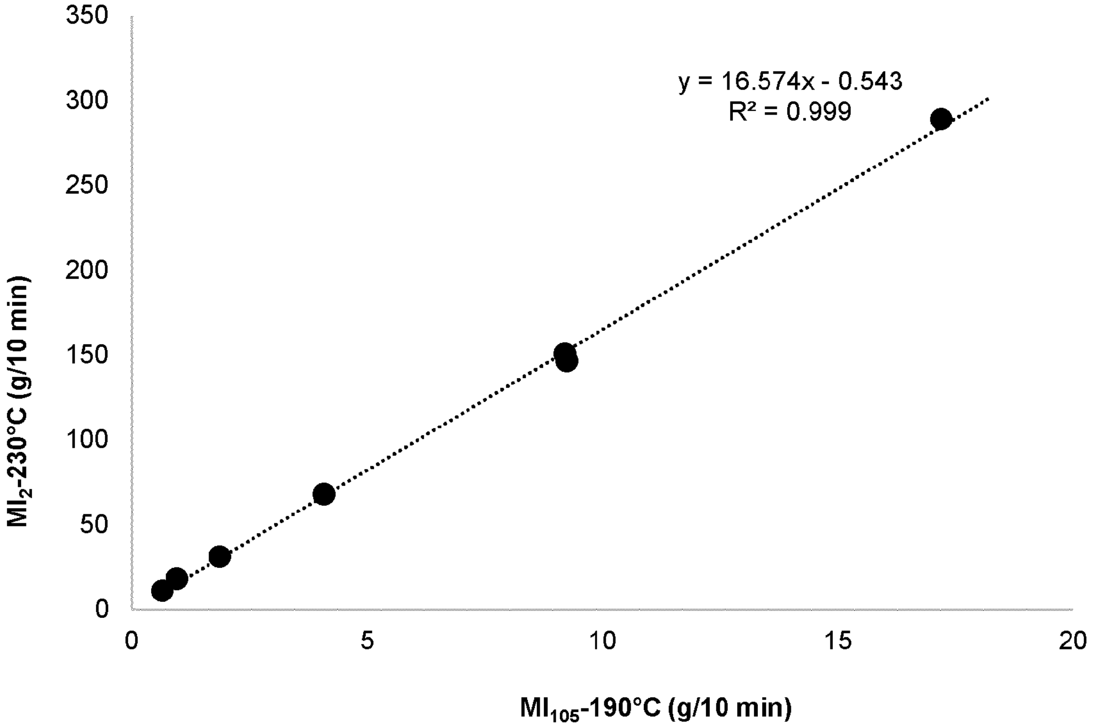
y = 16.574x - 0.543
R² = 0.999
350
300
250
200
150
100
50
0
0
5
10
15
20
MI2-230°C (g/10 min)
MI105-190°C (g/10 min)

FIG. 1

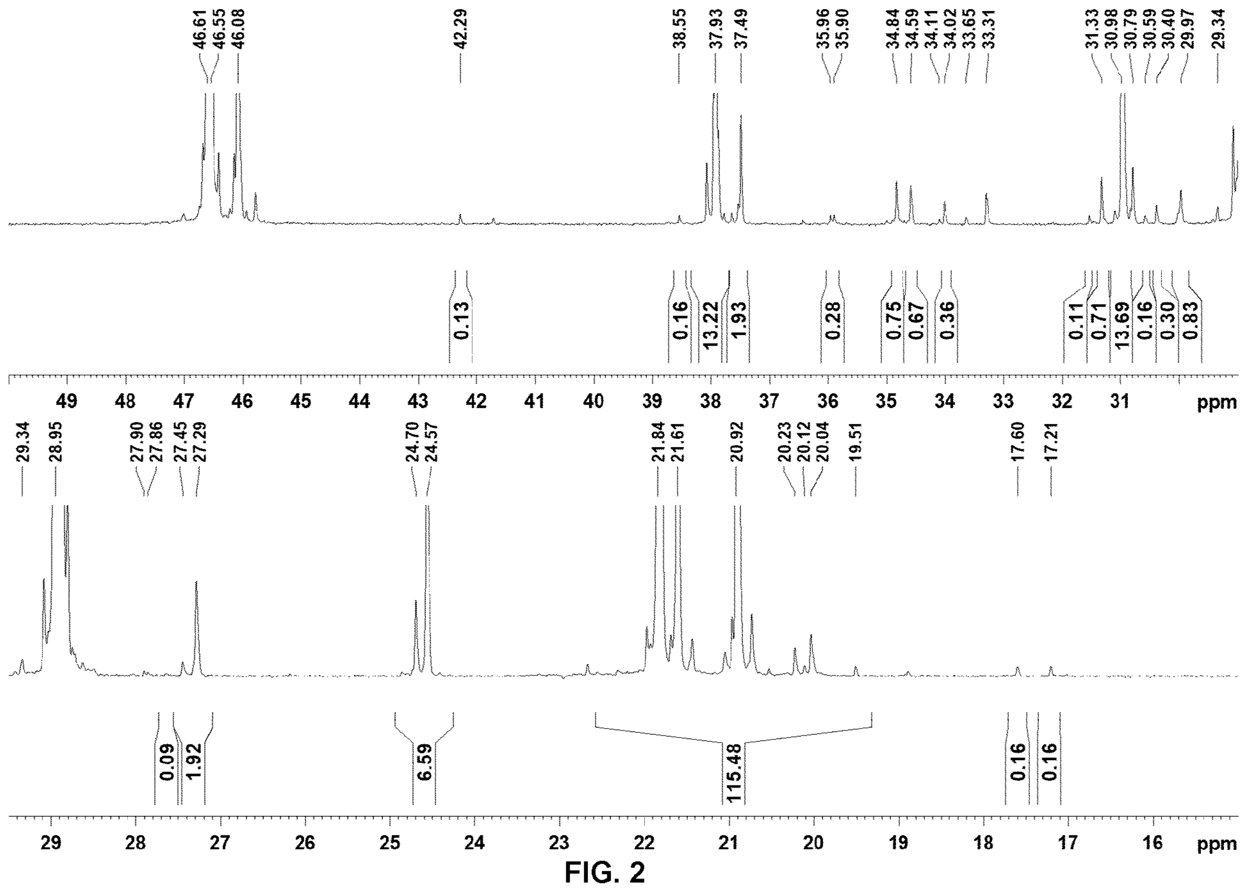
46.61
46.55
46.08
42.29
38.55
37.93
37.49
35.96
35.90
34.84
34.59
34.11
34.02
33.65
33.31
31.33
30.98
30.79
30.59
30.40
29.97
29.34
0.13
0.16
13.22
1.93
0.28
0.75
0.67
0.36
0.11
0.71
13.69
0.16
0.30
0.83
ppm
29.34
28.95
27.90
27.86
27.45
27.29
24.70
24.57
21.84
21.61
20.92
20.23
20.12
20.04
19.51
17.60
17.21
0.09
1.92
6.59
115.48
0.16
0.16
ppm

FIG. 2

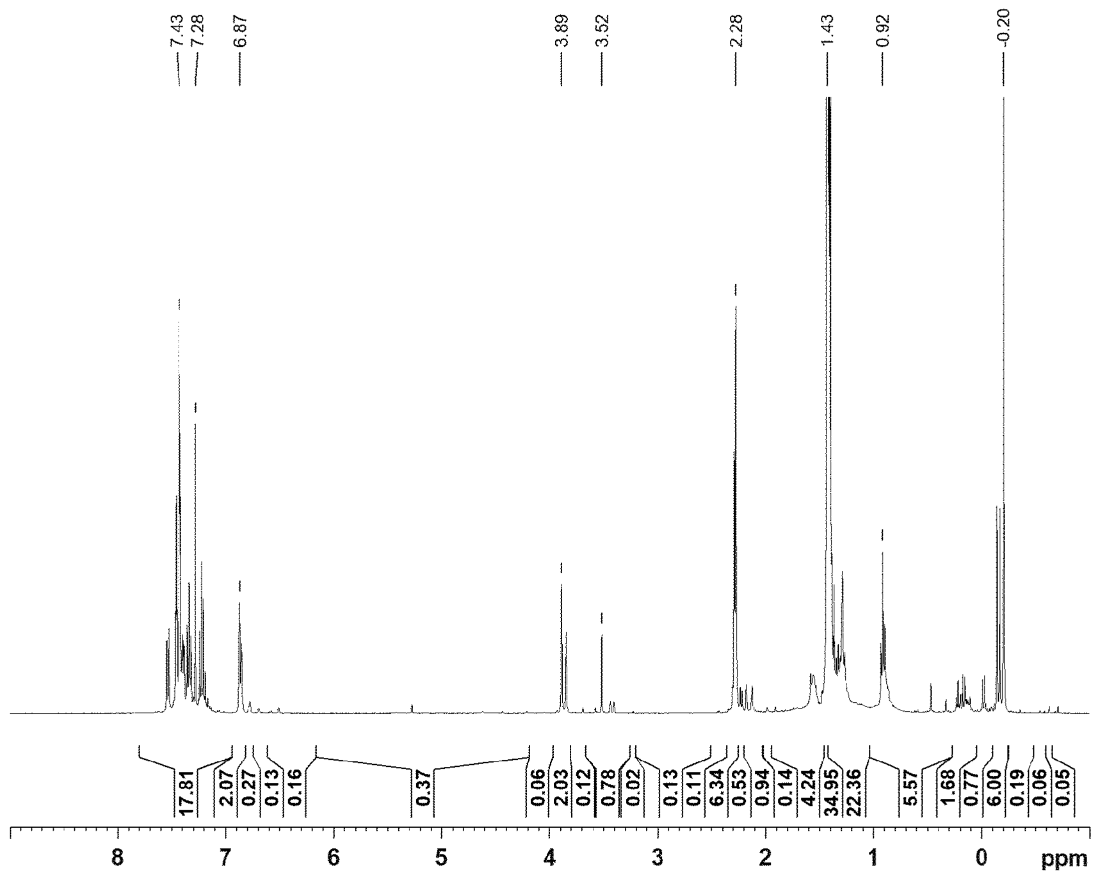

FIG. 3

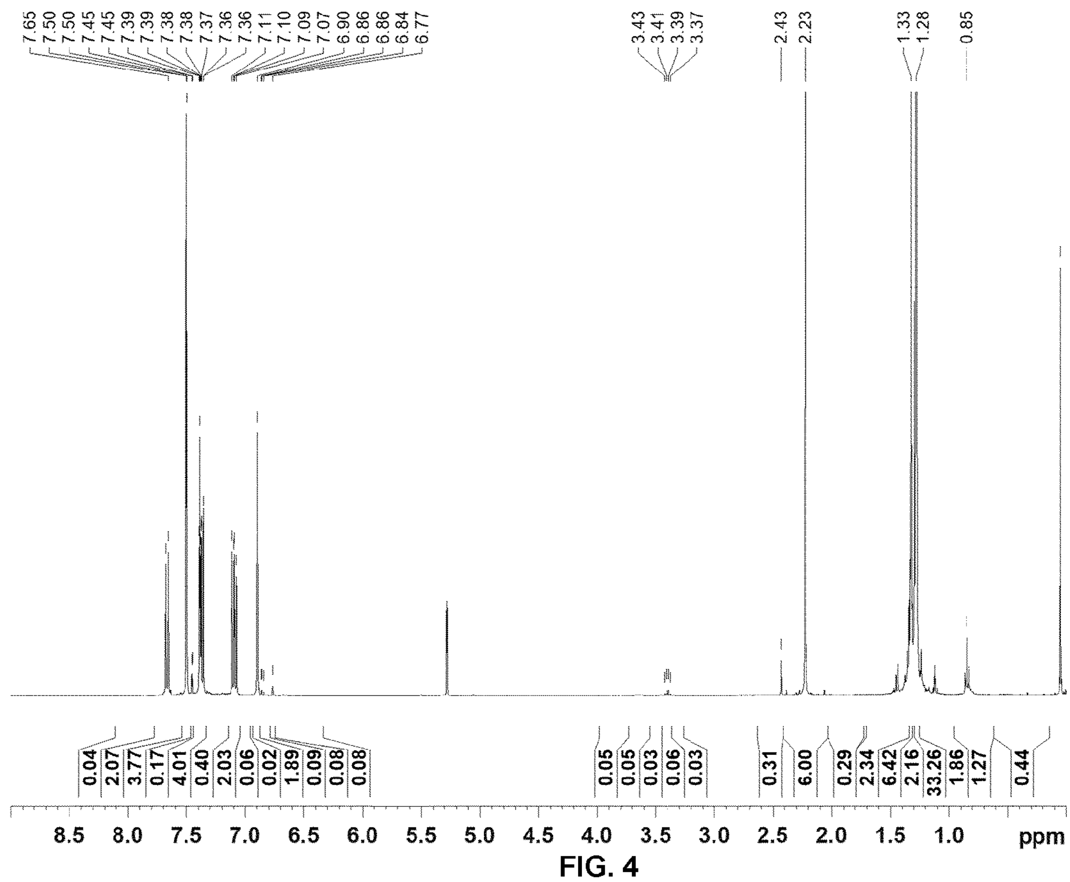

FIG. 4

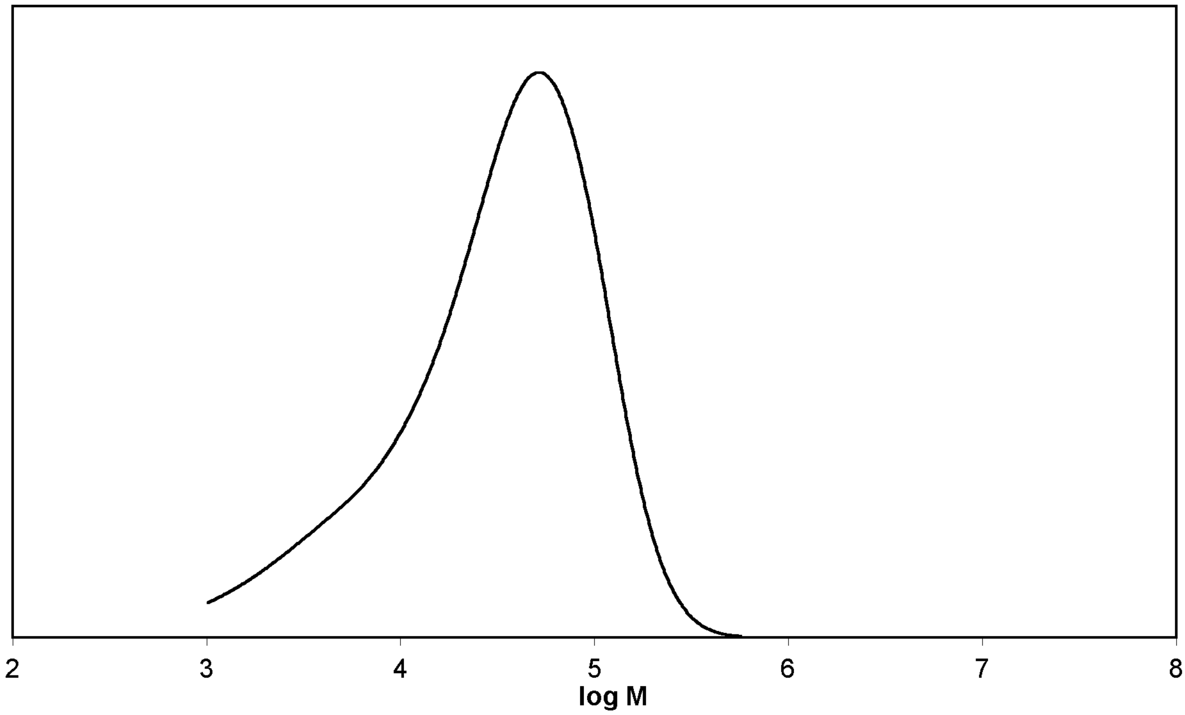


FIG. 5

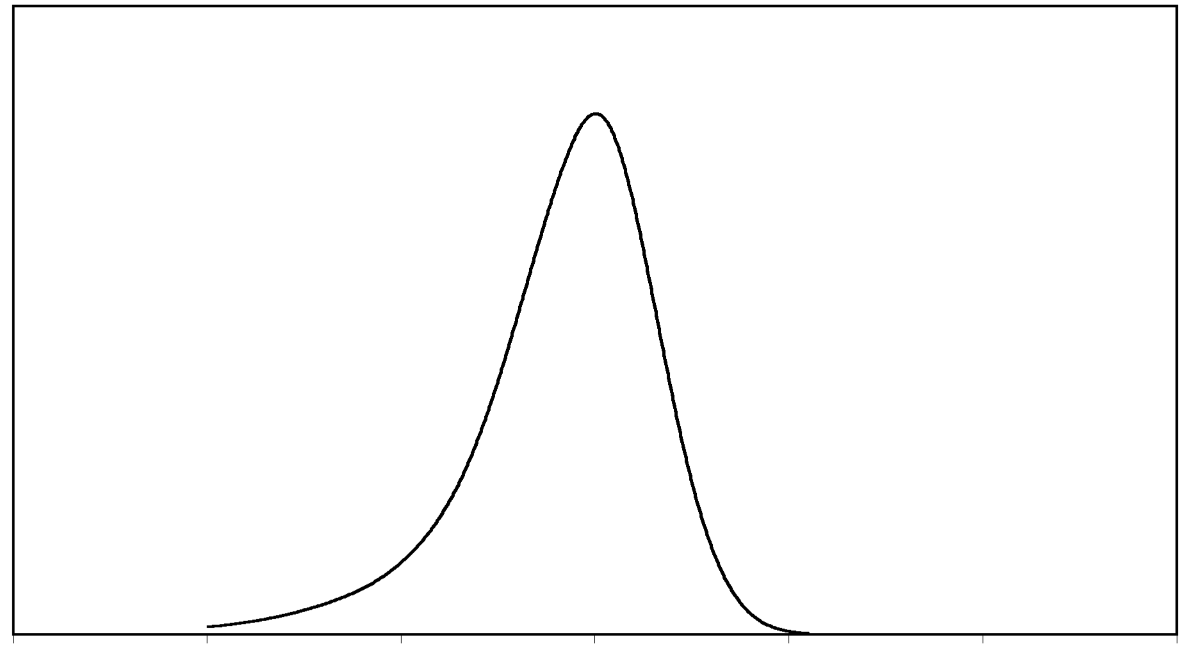


FIG. 6

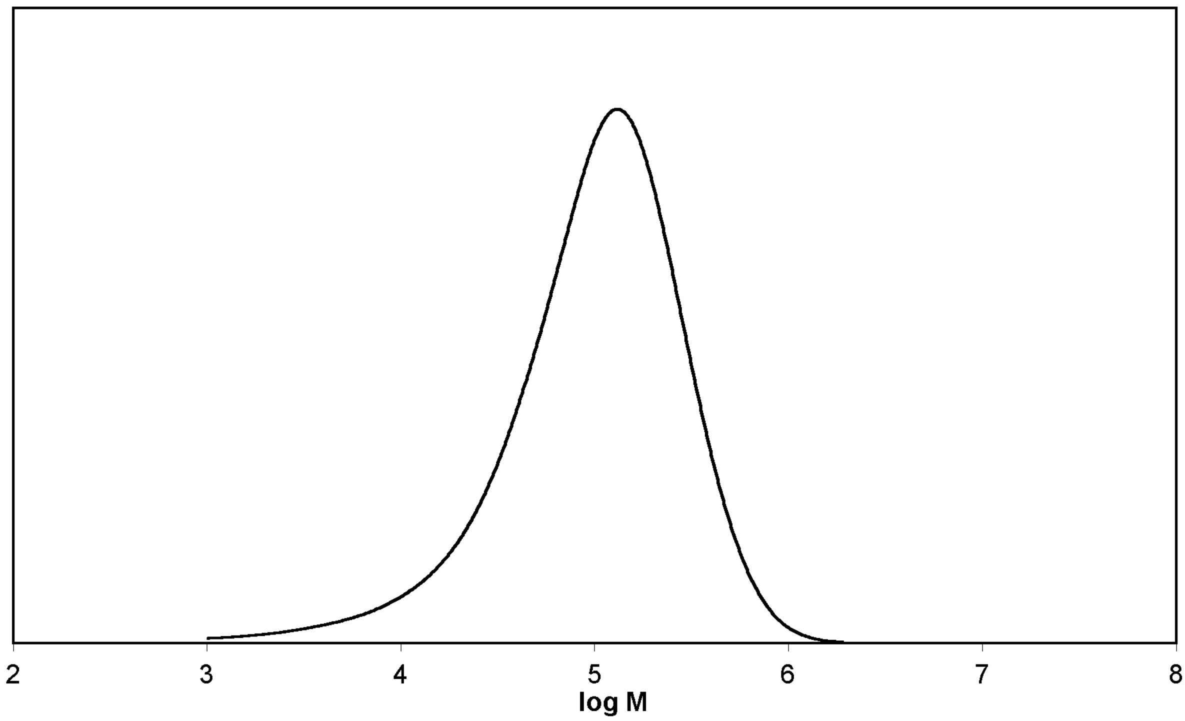


FIG. 7

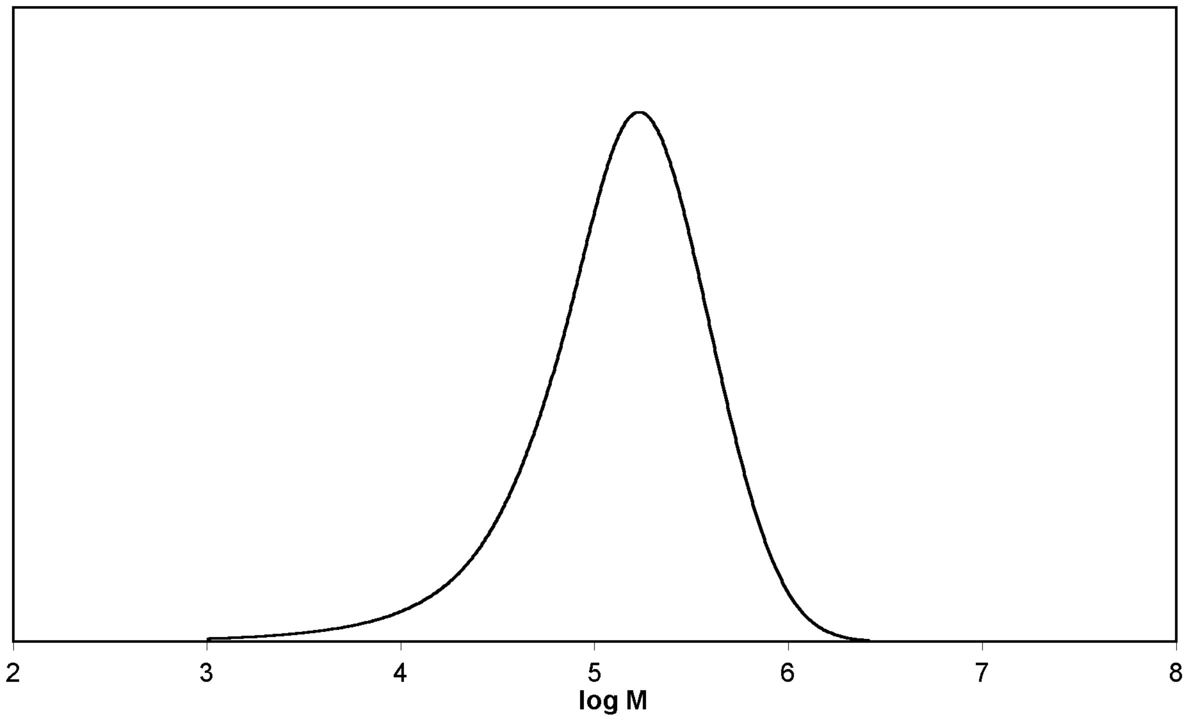


FIG. 8

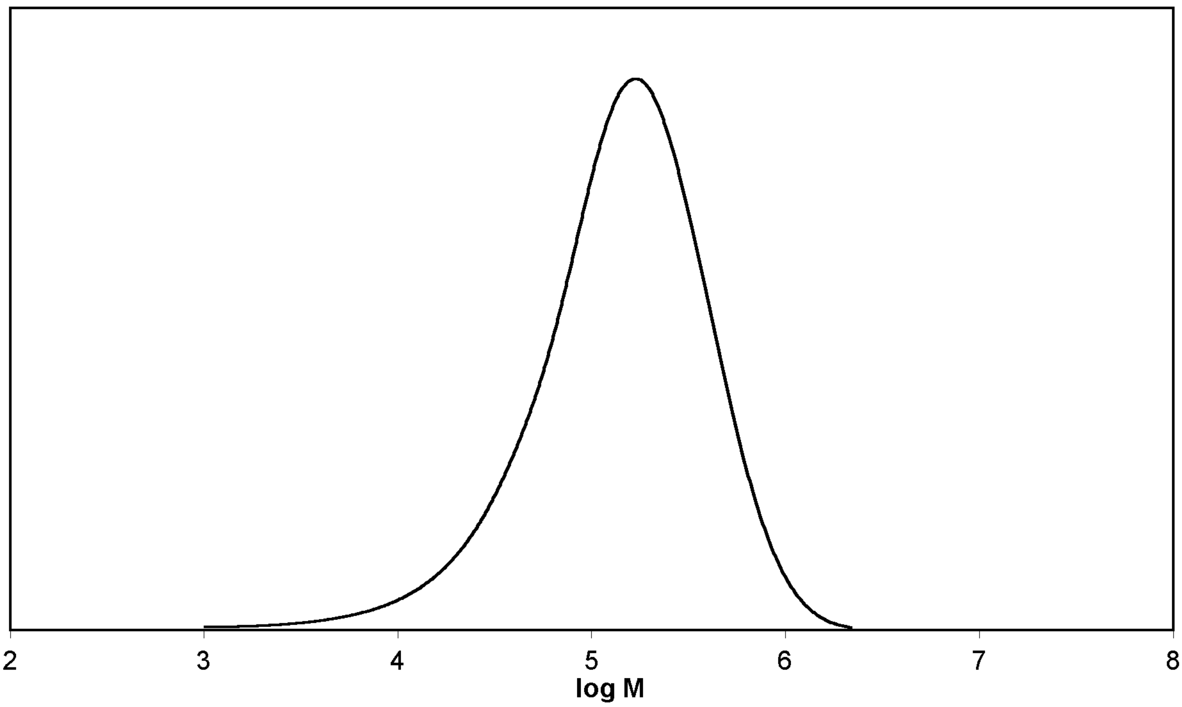


FIG. 9

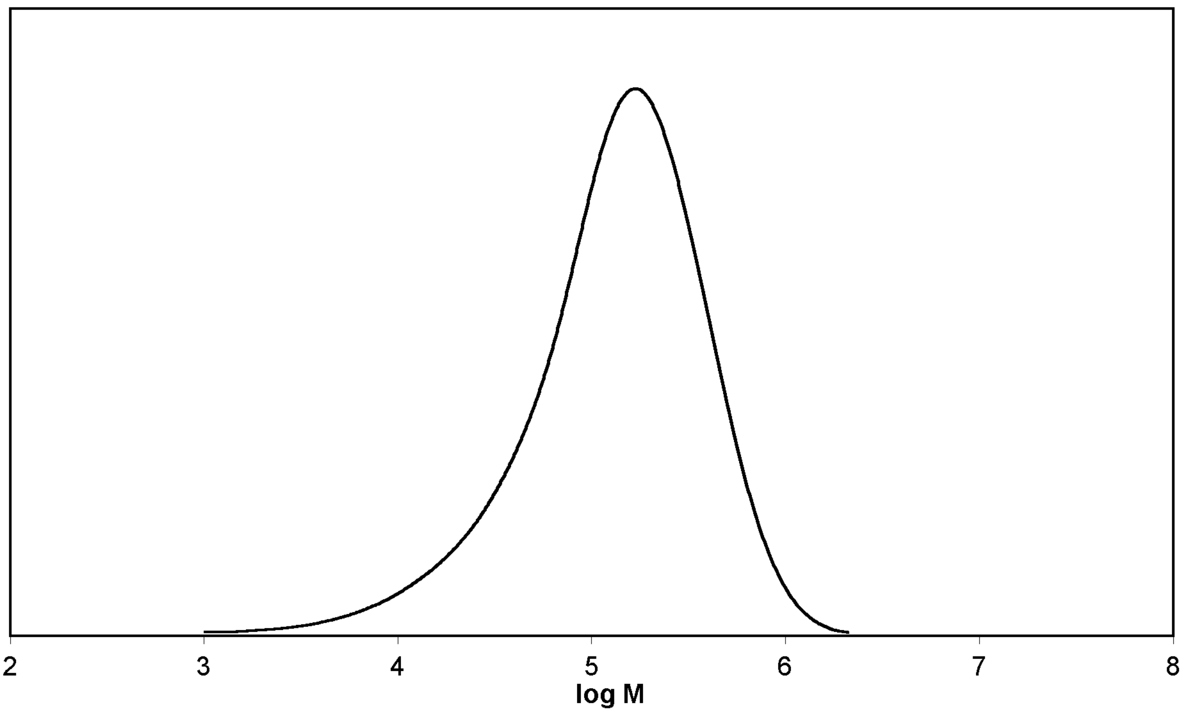


FIG. 10

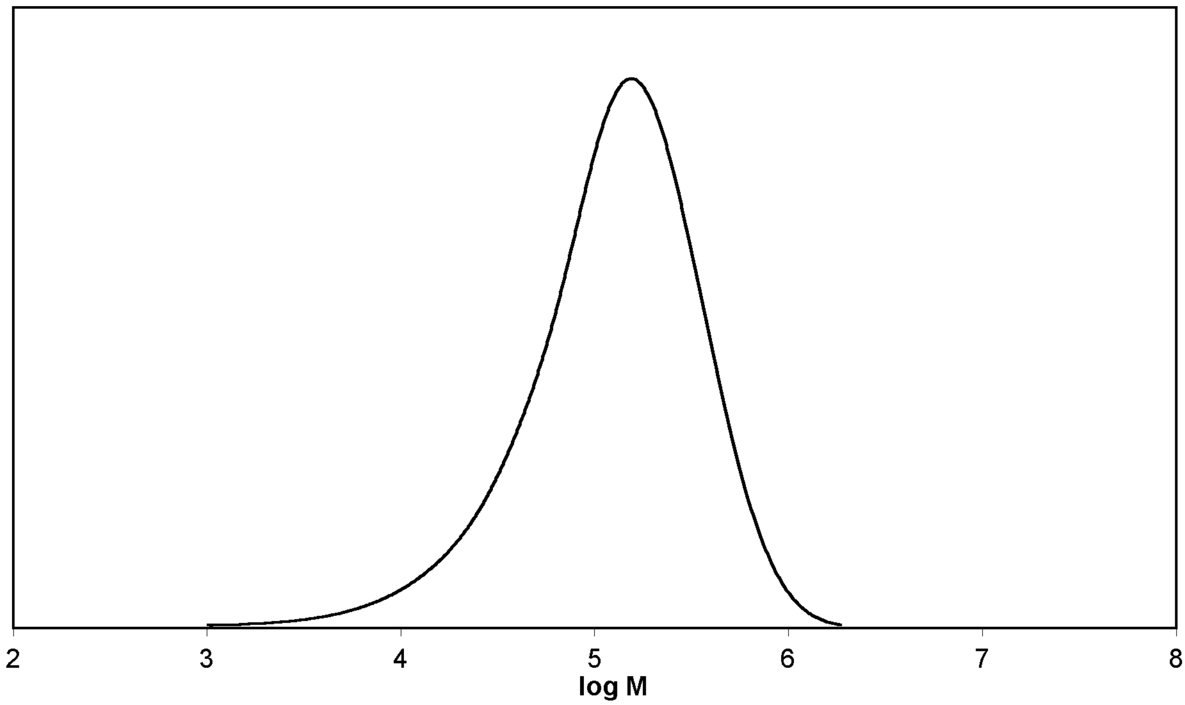


FIG. 11

Europäisches Patentamt
European Patent Office
Office européen des brevets

# EUROPEAN SEARCH REPORT

Application Number
EP 24 30 6608

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 118 598 726 A (CHINA CHEMICAL SCIENCE AND TECH RESEARCH CO LTD) 6 September 2024 (2024-09-06)<br>* Suzuki coupling of 4-bromo-2-methyl-1 H-indene with 3, 5-di(t-Bu)phenylboronic acid provides 4-(3',5'-di-t-butylphenyl)-2-methyl-1 H-indene (Cpd-3): see claim 1 and paragraphs [11] and [84] *<br>* "Cpd.3" is deprotonated with BuLi, the lithium salt reacted with dimethyldichlorosilane to give bis(4-(3,5-di-tert-butylphenyl)-2-methyl-1 H-inden-1-yl)dimethylsilane: see page 2, paragraph [26] and [98] *<br>* bis(4-(3,5-di-tert-butylphenyl)-2-methyl-1 H-inden-1-yl)dimethylsilane is deprotonated with an alkylmetal reagent, then treated with ZrCl4 to provide the ansa-Zr-complex III: see page 3, paragraph [39] *<br>* Use of the Zirconium catalyst in catalyst compositions for olefin polymerization: see claims 8 and 10 *<br>* A polymerization system and an olefin polymerization process using the catalyst composition: see claims 9-10 *<br>----- | 1-15 | INV.<br>C07F17/00<br>C07C1/32<br>C07F7/08<br>C07F5/02 |
| X | US 6 620 953 B1 (BINGEL CARSTEN [DE] ET AL) 16 September 2003 (2003-09-16)<br>* synthesis of rac-dimethylsilanediyl-bis(2-methylindenyl)-zirconium dichloride: see column 29, comparative example *<br>-----<br>-/-- | 9 | |

TECHNICAL FIELDS SEARCHED (IPC)

C07C
C07F

The present search report has been drawn up for all claims

1

| Place of search | Date of completion of the search | Examiner |
|---|---|---|
| The Hague | 21 February 2025 | Lange, Tim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 30 6608

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 532 687 A2 (BOREALIS AG [AT]) 12 December 2012 (2012-12-12) * synthesis of 7((3,5-di-tert-butylphenyl)-2-methylindene : see page 26, paragraph [194] * ----- | 1-7 | |
| | | | TECHNICAL FIELDS SEARCHED (IPC) |

1 The present search report has been drawn up for all claims

| Place of search | Date of completion of the search | Examiner |
|---|---|---|
| The Hague | 21 February 2025 | Lange, Tim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 30 6608

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2025

| Patent document cited in search report | Publication date | Patent family member(s) | Publication date |
|---|---|---|---|
| CN 118598726 A | 06-09-2024 | NONE | |
| US 6620953 B1 | 16-09-2003 | BR 9915708 A | 14-08-2001 |
| | | EP 1133504 A1 | 19-09-2001 |
| | | ES 2192408 T3 | 01-10-2003 |
| | | JP 2002530416 A | 17-09-2002 |
| | | US 6620953 B1 | 16-09-2003 |
| | | WO 0031091 A1 | 02-06-2000 |
| EP 2532687 A2 | 12-12-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9951344 A
- WO 0071554 A


### Non-patent literature cited in the description


- Plastics Additives Handbook. Hanser Publishers
- *CHEMICAL ABSTRACTS*, 888227-55-2
- **A. RAZAVI**. *Macromol. Symp.*, vol. 89, 345-367
- **H.N. CHENG** ; **J. EWEN**. *Makromol. Chem.*, 1989, vol. 190, 1931-1940